# EUROPEAN PATENT APPLICATION

(11) **EP 2 883 879 A1**
(43) Date of publication of application: **17.06.2015**
(21) Application number: 13382508.3
(22) Date of filing: 13.12.2013
(51) Int. Cl.: C07F 9/6574, C07B 43/04, C07D 209/08, C07D 401/12, C07D 209/14

(54) **Polymer supported phosphoric acids and use thereof as catalysts in the preparation of 3-indolylmethanamines**

(71) Applicant: Fundació Institut Català d'Investigació Química, 43007 Tarragona (ES)
(72) Inventor: Pericàs-Brondo, Miquel A., 08950 Esplugues de Llobregat (ES); Osorio Planes, Laura, 43001 Tarragona (ES); Rodríguez Escrich, Carles, 43001 Tarragona (ES)
(74) Representative: ZBM Patents - Zea, Barlocci & Markvardsen

(57) **Abstract**

The present invention relates to the field of catalysis, more particularly to the field of organocatalysis and to polymer supported chiral phosphoric acid catalysts. It also relates to the use of these compounds in the preparation of chiral 3-indolylmethanamines.

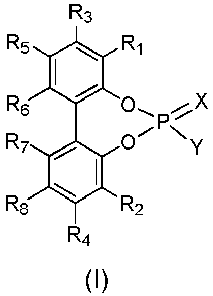

## Description

The present invention relates to the field of catalysis, more particularly to the field of organocatalysis and to polymer supported phosphoric acid catalysts. It also relates to the use of these compounds in the preparation of 3-indolylmethanamines.

### BACKGROUND ART

3-indolylmethanamines, and more particularly their chiral derivatives, are useful building blocks in the preparation of biologically active natural products and drugs, such as hydro-γ-carboline, gramine, aspidospermine alkaloids and pyrido[4,3-b]indole derivatives. Therefore, chiral 3-indolylmethanamines are of particular industrial interest in the preparation of pharmaceutical products or crop protecting agents.

Additionally, organocatalysis is emerging as a powerful tool for the preparation of asymmetric molecules and useful chiral building blocks. Since no metal is required for substrate activation in this particular field of catalysis, it is particularly attractive for applications in the life science industry, as it avoids the use of expensive and toxic metals. Organocatalysts are commonly robust organic small molecules, stable under air and operating under aerobic conditions. When containing chiral information, these compounds can provide high stereoselectivities in a broad range of reactions. They are usually used in rather large amounts and are difficult to recycle and isolate from the reaction product. In that sense, numerous homogeneous organocatalysts comprising a phosphoric acid moiety and useful in the preparation of 3-indolylmethanamines have been reported. Organic phosphoric acid catalysts have also been reported for other applications (e.g. asymmetric hydrogenation reactions).

In particular, Kang and co-workers reported a family of chiral organic phosphoric acids that are useful organocatalysts for the preparation of 3-indolylmethanamine derivatives by Friedel-Crafts condensation of indole derivatives onto imines formed from aldehydes. High yields and enantioselectivities were reported for the condensation of indole derivatives onto tosylated imines formed from aromatic aldehydes catalyzed by a broad range of chiral organic phosphoric acid catalysts, when the catalyst was used in 10 mol% amount and the reaction was carried out at low temperatures (e.g. -60 °C). Furthermore, it was shown that a decrease of the catalyst loading to 2 mol% provokes a decrease in the reaction yield and selectivity, indicating a low turnover number for this family of catalysts. Additionally, an excess of the indole derivative reagent, typically five equivalents with respect to the tosylated imines, was required so as to obtain high yields and selectivity and to avoid the formation of undesired side products.

On the other hand, the use of polymer supported organocatalysts is gaining interest, so as to allow a straightforward separation of a reaction product from the catalyst in the reaction mixture. Some efforts have been reported in the state of the art to support chiral organic phosphoric acids onto polymers.

In that sense, Rueping and co-workers have reported a polymer stick prepared by co-polymerization with styrene and divinylbenzene of a binaphthyl derived chiral phosphoric acid comprising a vinylphenyl radical either on positions 3 and 3' or on positions 6 and 6' of the naphthyl rings. The prepared compounds proved to be useful catalysts in the asymmetric hydrogenation of quinolone and benzoxazine derivatives with similar activity and selectivity as their non-polymer supported counterparts. The authors are however silent about the use of these compounds in the preparation of 3-indolylmethanamine compounds and in flow process applications. The morphology of the reported catalysts, i.e. polymer sticks, is also not suitable for continuous flow applications, as the available active surface of the catalyst is low.

In a different approach, Bleschke and co-workers have reported a binaphthyl derived phosphoric acid substituted at the 3 and 3' position with thienyl radicals, prompt to form small oligomers, thereby forming a microporous polymer comprising a chiral phosphoric acid. This compound proved to be active in the asymmetric reduction of benzoxazine derivatives, yet giving rise to such products in moderate enantioselectivity. The catalyst proved to be recyclable, achieving a total number of turnovers lower than 80. The authors are however silent about the use of this catalyst in continuous flow processes and in the preparation of 3-indolylmethanamine compounds.

From what is described in the state of the art, there is still the need for highly active, selective and recyclable chiral organic phosphoric acid catalysts supported onto a polymer and useful in the preparation of 3-indolylmethanamine derivatives, said catalysts being further suitable for continuous flow processes.

### SUMMARY OF THE INVENTION

The inventors have developed a family of compounds comprising chiral phosphoric acid derivatives linked to a polymeric support through a flexible linker, said compounds being useful as highly active and selective catalysts in the preparation of 3-indolylmethanamine compounds, and having the further advantage of being easily separated from the reaction mixture, recyclable without loss of activity or selectivity and suitable for continuous flow applications.

Thus, a first aspect of the invention refers to a chiral compound of formula (I) or a salt thereof 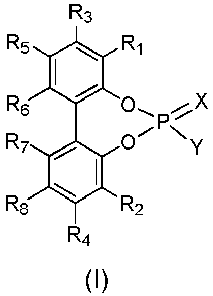 wherein:
X is S or O;
Y is selected from the group consisting of OH and a radical of formula-NHSO₂R₉, wherein R₉ is selected from the group consisting of (C₁-C₆)alkyl, (C₁-C₆)haloalkyl, phenyl and phenyl substituted with one or more groups selected from the group consisting of nitro, halogen and (C₁-C₆)alkyl;
R₁ and R₂ are each independently selected from the group consisting of (C₆-C₂₀ aryl); (C₅-C₂₀)heteroaryl; a radical of formula Si(R₁₀)₃ wherein R₁₀ is selected form the group consisting of phenyl and phenyl substituted with one or more group selected from the group consisting of nitro, halogen, (C₁-C₆)haloalkyl and (C₁-C₆)alkyl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, halogen and a radical of formula (II); and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, halogen and a radical of formula (II);
the radical of formula (II) is 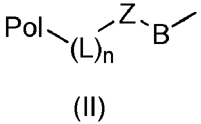 wherein
   B and L are each a biradical independently selected from the group consisting of (C₁-C₆)alkylene, phenylene, benzylene, -CH₂-C₆H₄-CH₂- and biphenylene ; n is 0 or 1;
   Z is a biradical linked to L and B selected from the group consisting of -O-,-S-, -CONR₁₁-, -SO₂NR₁₁-, -NR₁₁CO-, -NR₁₁SO₂- and -NR₁₁-; wherein R₁₁ is selected from the group consisting of H, (C₁-C₆)alkyl, phenyl and benzyl;
   Pol is a polymeric support;
R₃ and R₄ are each independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen;
each of the pairs R₅ and R₆, and R₇ and R₈, together with the carbon atoms to which they are attached, form a 6-membered carbocyclic ring A and a 6-membered carbocyclic ring A' respectively, said rings being independently saturated, partially unsaturated or aromatic and being optionally substituted with one or more groups selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen,
and the substituent of the position 6 and 6' of the fused ring system formed by the 6-membered ring A or the 6-membered ring A' and the adjacent phenyl ring in the compound of formula (I) is further selected from a radical of formula (II); and
with the proviso that the compound of formula (I) comprises at least one radical of formula (II).

A second aspect of the invention refers to a process for the preparation of the compound as defined in the first aspect of the invention, which comprises the steps of:
(i) contacting a polymeric support with a chiral compound of formula (IV) 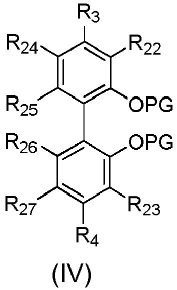
(ii) unprotecting the alcohols on the product obtained in (i);
(iii) treating the product obtained in (ii):
   with phosphorus oxychloride when in the compound of formula (I), X is O; or, alternatively,
   with thiophosphoryl chloride when, in the compound of formula (I), X is S;
(iv) treating the product obtained in (iii):
   with water or alternatively with an acid, when in the compound of formula (I), Y is OH; or, alternatively,
   with ammonia, when in the compound of formula (I), Y is a radical of formula-NSO₂R₉; and optionally
(v) when in the compound of formula (I), Y is a radical of formula -NSO₂R₉, then the process further comprises treating the compound obtained in (iv) with a compound of formula R₉SO₂Cl.
   wherein,
   in the compound of formula (IV):
   PG is an alcohol protecting group;
   R₂₂ and R₂₃ are independently selected from the group consisting of (C₆-C₂₀ aryl); (C₅-C₂₀)heteroaryl; a radical of formula Si(R₁₀)₃ wherein R₁₀ is selected from the group consisting of phenyl and phenyl substituted with one or more group selected from the group consisting of nitro, halogen, (C₁-C₆)haloalkyl and (C₁-C₆)alkyl; (C₆-C₂₀)aryl wherein one or more of the hydrogen atoms is replaced by one or more group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, halogen and a radical of formula (II'); and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, halogen and a radical of formula (II');
   the radical of formula (II') is 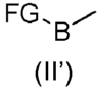
   B, R₃ and R₄ are as defined in any of the claims 1 to 9; and wherein
   each of the pairs R₂₄ and R₂₅, and R₂₆ and R₂₇, together with the carbon atoms to which they are attached, form a 6-membered carbocyclic ring D and a 6-membered carbocyclic ring D' respectively, said rings being independently saturated, partially unsaturated or aromatic and optionally substituted with one or more groups selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen, and the substituent of the position 6 and 6' of the fused ring system formed by the 6-membered ring D or the 6-membered ring D' and the adjacent phenyl ring in the compound of formula (IV) is further selected from a radical of formula (II'); and with the proviso that the compound of formula (IV) comprises at least one radical of formula (II'); and
wherein:
when in the compound of formula (I), Z is -O-, then the polymeric support comprises at least a terminal halogen group and in the compound of formula (IV), FG is hydroxyl;
or, alternatively,
when in the compound of formula (I), Z is -S-, then the polymeric support comprises at least a terminal halogen group and in the compound of formula (IV), FG is a -SH radical;
or, alternatively,
when in the compound of formula (I), Z is -CONR₁₁-, then the polymeric support comprises at least a terminal group selected from the group consisting of carboxylic acid and carboxylic acid chloride and in the compound of formula (IV), FG is a radical of formula NHR₁₁;
or, alternatively,
when in the compound of formula (I), Z is -NR₁₁CO-, then the polymeric support comprises at least a terminal group of formula NHR₁₁ and in the compound of formula (IV), FG is a group selected from the group consisting of carboxylic acid and carboxylic acid chloride;
or, alternatively,
when in the compound of formula (I), Z is -SO₂NR₁₁-, then the polymeric support comprises at least a terminal sulfonyl chloride group and in the compound of formula (IV), FG is a radical of formula NHR₁₁;
or, alternatively,
when in the compound of formula (I), Z is -NR₁₁SO₂-, then the polymeric support comprises at least a terminal group of formula NHR₁₁ and in the compound of formula (IV), FG is a sulfonyl chloride group;
or, alternatively,
when in the compound of formula (I), Z is -NR₁₁-, then the polymeric support comprises at least a terminal halogen group and in the compound of formula (IV), FG is a radical of formula NHR₁₁; or, alternatively, the polymeric support comprises at least a terminal group of formula NHR₁₁ and in the compound of formula (IV), FG is halogen;
being R₁₁ selected from the group consisting of H, (C₁-C₆)alkyl, phenyl and benzyl.

When used as catalysts, the compounds of the invention are particularly active and selective in the preparation of 3-indolylmethanamine compounds by Friedel-Crafts condensation of indole derivatives onto imines. The compounds of the invention can be recycled without loss of activity and selectivity and are suitable for the preparation of 3-indolylmethanamine compounds in continuous flow conditions. Flow chemistry is increasingly perceived as a replacement technology for traditional batch processing. Thus, many negative issues associated to batch processing, such as solvent waste (mainly associated to work-up), thermal risk, and problems associated to scale-up are completely avoided in flow processing.

Thus, a third aspect of the invention refers to the use of the compound according to the first aspect of the invention as a catalyst.

The catalysts of the invention can be used under continuous flow conditions, i.e. by the injection into a reactor comprising the compound of the invention of a solution of reactants.

A fourth aspect of the invention relates to a process for the preparation of a 3-indolylmethanamine of formula (XI) 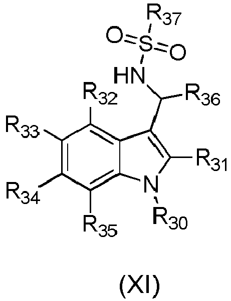 which comprises contacting a compound of formula (IX) with a compound of formula (X) in the presence of a compound of formula (I) as defined in the first aspect of the invention 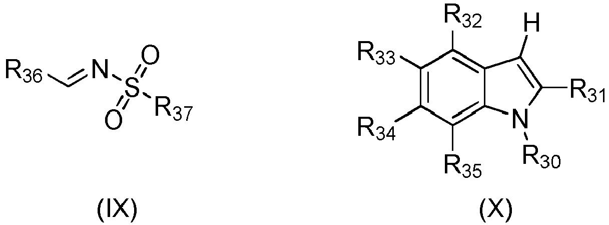 wherein:
R₃₀ is selected from the group consisting of hydrogen, (C₁-C₆)alkyl, (C₁-C₆)haloalkyl, (C₁-C₆)alkylcarbonyl, (C₁-C₆)alkyloxycarbonyl, and benzyl;
R₃₁, R₃₂, R₃₃, R₃₄ and R₃₅ are independently selected from the group consisting of hydrogen, cyclic ring, (C₆-C₂₀)aryl, (C₅-C₂₀)heteroaryl, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, halogen, (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, and halogen;
R₃₆ is selected from the group consisting of hydrogen; (C₁-C₆)alkyl; (C₁-C₆)haloalkyl; cyclic ring; (C₆-C₂₀ aryl); (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R₃₇) and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R₃₇) and halogen; and
R₃₇ is selected from the group consisting of (C₁-C₆)alkyl; (C₁-C₆)haloalkyl; cyclic ring; benzyl; (C₆-C₂₀ aryl); (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen;
being cyclic ring a saturated, or partially unsaturated, 3- to 7-membered ring, or 3- to 7-membered ring bridged or fused with one or more 5 to 12 membered ring, saturated, or partially unsaturated, or aromatic, the members of the rings being selected from C, CH, CH₂, O, S, N and NH; being one or more of the hydrogen atoms of the members optionally substituted by a radical selected from the group consisting of (C₁-C₆)alkyl, (C₁-C₆)haloalkyl, halogen, (C₁-C₆)alkoxy, nitro, cyano, (C₁-C₆)alkylcarbonyl, and (C₁-C₆) alkyloxycarbonyl.

As mentioned above, the compounds of the invention comprise a flexible linker. Unlike the catalysts reported in the prior art, the compounds of the invention allow for the preparation of 3-indolylmethanamine compounds with good yields and enantioselectivities, even at room temperature and when a minor excess of the indole reagent is used. The compounds of the invention are also more active catalysts than those described in the prior art and can be used at lower loadings under flow conditions thanks to their robustness, therefore giving rise to high turnover numbers. Without being bound to theory, it is believed that the use of a flexible linker between the polymeric support and the chiral phosphoric acid moiety allows for a more flexible spatial arrangement of the system, thus giving rise to a synergistic effect between the asymmetric induction provided by the chiral phosphoric acid moiety and the hydrophobic environment created by the polymeric support.

### BRIEF DESCRIPTION OF THE DRAWINGS

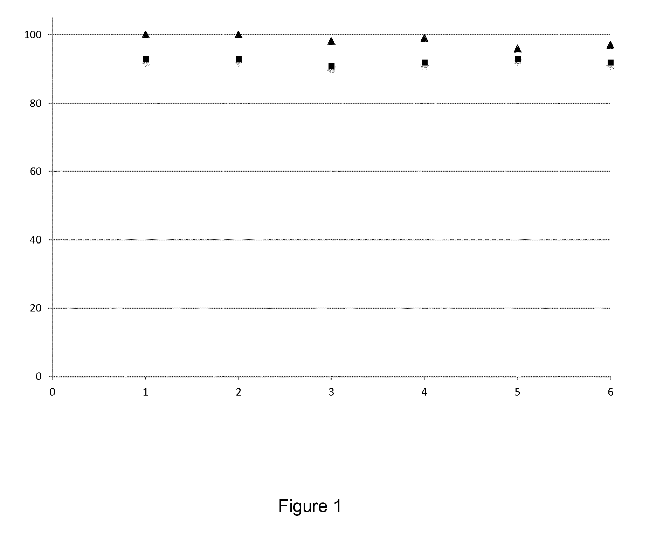

Fig. 1 shows the evolution over time of the conversion, represented as triangles, and enantiomeric excess, represented as squares, both expressed as percentages, of aliquots of solution taken at the outlet of a reactor comprising the compounds of the invention and fed with a solution of reagents to produce (*S*)-N-((1H-indol-3-yl)(*p*-tolyl)methyl)-4-methylbenzenesulfonamide. Y axis is expressed in percentage and X axis represents the time, expressed in hours.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set-out definition provides a broader definition.

The term "chiral compound" refers to a compound which contains chiral information. In the context of the invention, the compound of formula (I) is chiral when the rotation around the bond linking the two phenyl rings comprised in the compound of formula (I) is energetically demanding, to such an extent allowing for the formation of enantiomers.

In the context of the invention, a "salt of a chiral compound of formula (I)" refers to a compound of formula (I) wherein the acidic hydrogen atom born by the oxygen or nitrogen atom of the Y radical has been replaced by a metal selected from the group consisting of indium, aluminium, manganese, copper, ruthenium, rhodium, palladium, silver, lithium, sodium, potassium, rubidium, caesium, magnesium, calcium, barium, beryllium and mixtures thereof.

The term "alkyl" refers to a saturated straight or branched hydrocarbon chain which contains the number of carbon atoms specified in the description or claims. Thus, the term "(C₁-C₆)alkyl" refers to a saturated straight, or branched hydrocarbon chain containing from 1 to 6 carbon atoms. Examples include the group methyl, ethyl, propyl, isopropyl, butyl, isobutyl, *sec*-butyl, *tert*-butyl, n-pentyl, *neo*-pentyl and *n*-hexyl.

The term "halogen", also referred to as halo, refers to fluoro, chloro, bromo or iodo.

The term (C₁-C₆)haloalkyl refers to a group resulting from the replacement of one or more hydrogen atoms from a (C₁-C₆)alkyl group with one or more, preferably from 1 to 6, halogen atoms, which can be the same or different. Examples include, among others, trifluoromethyl, fluoromethyl, 1-chloroethyl, 2-chloroethyl, 1-fluoroethyl, 2-fluoroethyl, 2-bromoethyl, 2-iodoethyl, 2,2,2-trifluoroethyl, pentafluoroethyl, 3-fluoropropyl, 3-chloropropyl, 2,2,3,3-tetrafluoropropyl, 2,2,3,3,3-pentafluoropropyl, heptafluoropropyl, 4-fluorobutyl, and nonafluorobutyl.

The term "(C₆-C₂₀)aryl" as used herein, whether used alone or as part of another group, is defined as a radical derived from one of the known ring systems with 1-4 phenyl rings, having from 6 to 20 carbon atoms wherein each one of the phenyl rings forming said ring system is isolated or, partially or totally fused.

Non-limitative examples include phenyl, naphthyl, phenantryl, anthracenyl, biphenylyl and phenylylnaphthalene.

The term "(C₅-C₂₀)heteroaryl" as used herein, whether used alone or as part of another group, is defined as a radical derived from one of the known ring systems with 1-4 5-membered or 6-membered aromatic rings, and having from 5 to 20 atoms, each member being independently selected from the group consisting of C, O, N, NH, S and CH, and wherein each one of the rings forming said ring system is isolated or, partially or totally fused. Non-limitative examples include pyrrolyl, furyl, thiophenyl, pyrazolyl, indolyl, benzofuryl, benzothiofuryl, quinolyl, isoquinolyl, naphtyridyl, imidazolyl, triazolyl, tetrazolyl, oxazolyl, isoxazolyl, thiazolyl, isothiazolyl, dithiolanyl, pyridyl, , pyridazinyl, pyrimidinyl, and pyrazinyl.

In preferred embodiments of the invention, when one or more of the hydrogen atoms of (C₆-C₂₀)aryl, or phenyl, or cyclic ring, or (C₅-C₂₀)heteroaryl is replaced by a group, then the term "one or more" refers to "from 1 to 6". In more preferred embodiments the term "one or more" refers to "from 1 to 3".

The term "(C₁-C₆)alkoxy" refers to an alkyloxy group, or a (C₁-C₆)alkyl-O-radical, having from 1 to 6 carbon atoms, the alkyl moiety having the same meaning as previously defined. Examples include, among others, methoxy, ethoxy, propoxy, isopropoxy, tert-butoxy, pentoxy and hexyloxy.

The term "(C₁-C₆)alkylcarbonyl" refers to a saturated straight or branched alkyl chain which contains from 1 to 6 carbon atoms, where the carbon atom is appended to the alkyl chain through a carbonyl group. Examples include acetyl, 1-oxopropyl, 1-oxoisobutyl and 1-oxobutyl.

The term "(C₁-C₆)alkyloxycarbonyl" refers to a saturated straight or branched alkyl chain which contains from 1 to 6 carbon atoms, where the carbon atom is appended to the alkyl chain through an oxycarbonyl group (-OCO). Examples include methylcarboxy, ethylcarboxy, propylcarboxy, isopropylcarboxy, butylcarboxy, *tert*-butylcarboxy, pentylcarboxy, and hexylcarboxy.

The term "(C₁-C₆)alkylcarbonyloxy" refers to a saturated straight or branched alkyl chain which contains from 1 to 6 carbon atoms, where the carbon atom is appended to the alkyl chain through an carbonyloxy group (-COO). Examples include methanoyloxy, ethanoyloxy, propanoyloxy, isopropanoyloxy, butanoyloxy, *tert*-butanoyloxy, pentanoyloxy, and hexanoyloxy.

The term "(C₁-C₆)alkylene" refers to a diradical resulting from the removal of one hydrogen atom of (C₁-C₆)alkyl.

The term "polymeric support" is art-recognized and refers to a soluble or insoluble polymer onto which the active unit is anchored directly or through a linker. Many suitable polymeric supports are known, and include soluble polymers such as polyethylene glycols or polyvinyl alcohols, as well as insoluble polymers such as polystyrene resins. In the context of the invention, the polymeric support can be porous. Non-limitative examples of polymeric support include polystyrene, cross-linked polystyrene with for example divinylbenzene, polyacrylate, polyacrylamide, polyvinylpyrrolidinone, polysiloxane, polybutadiene, polyisoprene, polyalkane, polyoxazoline, polyether, and mixtures and co-polymers thereof.

In the context of the invention, in the compounds of formula (I) and (IV), the position 6 or 6' of a fused ring system formed by a 6-membered ring and the adjacent phenyl ring refers to the position, available for substitution, according to IUPAC rules, numbered 6 or 6' as defined below, and wherein, in the compound of formula (I), the moiety of formula O₂PXY is bound at positions 2 and 2', R₁ and R₂ at positions 3 and 3' respectively, and R₃ and R₄ at positions 4 and 4' respectively; or, alternatively, in the compound of formula (IV), the moiety of formula OPG is bound at positions 2 and 2', R₂₂ and R₂₃ at positions 3 and 3' respectively, and R₃ and R₄ at positions 4 and 4' respectively:

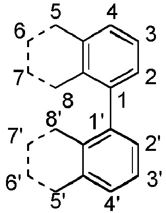

The term "alcohol protecting group" is known in the art, and refers to a chemical moiety or group which protects or prevents an alcohol moiety or group from participating with or interfering with one or more chemical synthetic steps and its removal restores the moiety to its original active state. The term protecting group as used herein refers to those groups intended to protect against undesirable reactions during synthetic procedures. Such protecting groups are well known to those skilled in the art. Examples of alcohol protecting groups can be found in Wuts et al.,"Greene's Protective Groups in Organic Chemistry", (Wiley, 4th ed. 2007, p.16-23). Protecting groups can be removed with inter alia acid, base, fluoride ions, hydrogenation, metals such as zinc as well as by numerous other methods which are well known in the art. One of ordinary skill in the art can readily choose an appropriate protecting group to facilitate synthetic reactions according to method aspects of the present invention without engaging in undue experimentation. Further non-limitative examples of alcohol protecting groups include: tri(C₁-C₆)alkylsilyl, *tert*-butyldiphenylsilyl (TBDPS), benzyl (Bn), tetrahydropyranyl (THP), methoxymethyl (MOM), (C₁-C₆)alkyl, *p*-methoxybenzyl (PMB), benzoyl (Bz), and acetyl (Ac).

In the context of the invention, the term "continuous flow process" refers to a process wherein the reagents of the process, either in solution (i.e. dissolved into a solvent) or neat, are constantly fed into a reactor in the right proportions, thus allowing for the continuous production of the product. When a catalyst is present, the reactor further comprises the catalyst, preferably as an insoluble solid.

In the context of the invention, the term "yield", refers to the ratio, expressed as a percentage, of the molar amount of a 3-indolylmethanamine produced to the molar amount of compound of formula (IX) engaged in the process of preparation of the 3-indolylmethanamine.

The "enantiomeric excess" or "*ee*" is a measure of the excess of one enantiomer over a racemic mixture of a chiral compound, which is commonly expressed as a percentage. Enantiomeric excess is defined as the absolute difference between the mole fraction of each enantiomer [ee = F(+)- F(-)]. If the moles of each enantiomer are known, the percent enantiomeric excess can be determined by the following formula: *ee* = ((R-S)/(R+S)) x 100, where R and S are the respective molar fractions of enantiomers in the mixture such that R+S=1.

The terms "3-indolylmethanamines" or "3-indolylmethanamine compounds" or "3-indolylmethanamine derivatives" are used interchangeably and refer to a compound comprising the moiety resulting from the removal of 1 to 9 hydrogen atoms of the compound of formula

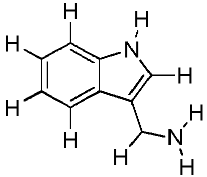

In the context of the invention, and when referred to as a substituent, the term "cyclic ring" refers to a saturated, or partially unsaturated, 3- to 7-membered ring, or 3- to 7-membered ring bridged or fused with one or more 5 to 12 membered ring, saturated, or partially unsaturated, or aromatic, the members of the rings being selected from C, CH, CH₂, O, S, N and NH; being one or more of the hydrogen atoms of the members optionally substituted by a radical selected from the group consisting of (C₁-C₆)alkyl, (C₁-C₆)haloalkyl, halogen, (C₁-C₆)alkoxy, nitro, cyano, (C₁-C₆)alkylcarbonyl, and (C₁-C₆) alkyloxycarbonyl. Non-limitative examples include cyclopentyl, cyclohexyl, cyclopropyl, cyclobutyl, piperidinyl, fluorenyl, tetrahydrofuranyl, decalinyl, pyrollidinyl, tetrahydrothiophenyl and the like.

As mentioned above, the inventors have developed polymer supported organic phosphoric acid catalysts useful in the preparation of 3-indolylmethanamine compounds. The catalysts of the invention are robust, do not deactivate easily and can be separated from the reaction mixture by, for example, filtration or centrifugation. In particular, the inventors have found that the catalysts of the invention are useful in continuous flow applications, thanks to their high activity, selectivity and robustness. In contrast with the catalysts described in the state of the art for the preparation of 3-indolylmethanamine compounds, the catalysts of the invention provide higher reactivity and enantioselectivity and can be easily recycled and regenerated without loss of activity. The anchoring of the organic phosphoric acids onto a polymeric support via a flexible linker, as described in the catalysts of the invention, surprisingly enhances the activity of the catalyst and improves the chiral induction of the process. As a result, 3-indolylmethanamine compounds can be prepared in the presence of a minor excess of the indole reagent, at lower catalyst loadings, and with higher enantiomeric excesses. Furthermore, the catalysts of the invention can be easily recycled and regenerated and are thus suitable for continuous flow applications. The compounds of formula (I) are also robust towards moisture and oxygen and can be easily handled.

In a preferred embodiment of the first aspect of the invention, X is O.

In a preferred embodiment of the first aspect of the invention, Y is selected from the group consisting of -OH and a radical of formula -NSO₂R₉ wherein R₉ is selected from the group consisting of methyl, trifluoromethyl, phenyl and tolyl. In a more preferred embodiment, Y is -OH or -NHSO₂CF₃. In an even more preferred embodiment, Y is -OH.

In a preferred embodiment of the first aspect of the invention, R₃ and R₄ are independently selected from the group consisting of hydrogen, (C₁-C₆)alkyl, (C₁-C₆)alkyloxy, (C₁-C₆)haloalkyl, nitro, cyano and halogen. In a more preferred embodiment, R₃ and R₄ are each independently selected from the group consisting of hydrogen, methyl, ethyl, methoxy, trifluoromethyl, nitro, cyano and halogen. In an even more preferred embodiment, R₃ and R₄ are each hydrogen.

In a preferred embodiment of the first aspect of the invention, R₁ and R₂ are each independently selected from the group consisting of (C₆-C₂₀ aryl); (C₅-C₂₀)heteroaryl; a radical of formula Si(R₁₀)₃ wherein R₁₀ is selected from the group consisting of phenyl and phenyl substituted with one or more group selected from the group consisting of nitro, halogen, (C₁-C₆)haloalkyl and (C₁-C₆)alkyl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, and halogen.

In a more preferred embodiment, R₁ and R₂ are each independently selected from the group consisting of phenyl, naphthyl, anthracenyl, phenantrenyl, biphenylyl, phenylylnaphthalene, furyl, pyridyl, thiophenyl, pyrrolyl, indolyl, benzofuryl, benzothiofuryl, triazolyl, pyrimidyl, quinolyl, isoquinolyl, naphthyridyl and a radical of formula Si(R₁₀)₃ wherein R₁₀ is selected from the group consisting of phenyl and phenyl substituted with (C₁-C₆)alkyl, and wherein the phenyl, naphthyl, anthracenyl, phenantrenyl, biphenylyl, phenylylnaphthalene, furyl, pyridyl, thiophenyl, pyrrolyl, indolyl, indenyl, benzofuryl, benzothiofuryl, triazolyl, quinolyl, isoquinolyl, naphthyridyl and pyrimidyl groups are optionally substituted with one or more groups selected from the group consisting of nitro, (C₁-C₆)alkyl, (C₁-C₆)haloalkyl, phenyl and halo.

In another embodiment, R₁ and R₂ are each independently selected from the group consisting of phenyl, naphthyl, anthracenyl, phenantrenyl and a radical of formula Si(R₁₀)₃ wherein R₁₀ is selected from the group consisting of phenyl and phenyl substituted with (C₁-C₆)alkyl, and wherein the phenyl, naphthyl, anthracenyl, phenantrenyl groups are optionally substituted with one or more groups selected from the group consisting of nitro, (C₁-C₆)alkyl, (C₁-C₆)haloalkyl, phenyl and halo.

In another embodiment, R₁ and R₂ are each independently selected from the group consisting of phenyl, naphthyl, anthracenyl, phenantrenyl and a radical of formula Si(R₁₀)₃ wherein R₁₀ is selected from the group consisting of phenyl and phenyl substituted with (C₁-C₆)alkyl, and wherein the phenyl, naphthyl, anthracenyl, phenantrenyl groups are optionally substituted with one or more groups selected from the group consisting of (C₁-C₆)alkyl, (C₁-C₆)haloalkyl and phenyl.

In another embodiment, R₁ and R₂ are each independently selected from the group consisting of phenyl, naphthyl, anthracenyl, phenantrenyl and a radical of formula Si(R₁₀)₃ wherein R₁₀ is selected from the group consisting of phenyl and phenyl substituted with (C₁-C₆)alkyl, and wherein the phenyl, naphthyl, anthracenyl, phenantrenyl groups are optionally substituted with one to three groups selected from the group consisting of (C₁-C₆)alkyl, (C₁-C₆)haloalkyl and phenyl.

In another embodiment, R₁ and R₂ are each independently selected from the group consisting of phenyl, naphth-2-yl, anthracen-9-yl, phenantren-9-yl and a radical of formula Si(R₁₀)₃ wherein R₁₀ is selected from the group consisting of phenyl and 4-tert-butylphenyl, and wherein the phenyl, naphth-2-yl, anthracen-9-yl, phenantren-9-yl groups are optionally substituted with one or more groups selected from the group consisting of (C₁-C₆)alkyl, (C₁-C₆)haloalkyl and phenyl. In an even more preferred embodiment, R₁ and R₂ are each independently selected from the group consisting of phenyl, naphth-2-yl, anthracen-9-yl, phenantren-9-yl and a radical of formula Si(R₁₀)₃ wherein R₁₀ is selected from the group consisting of phenyl and 4-*tert*-butylphenyl, and wherein the phenyl group is optionally substituted with one or more groups selected from the group consisting of methyl, trifluoromethyl, iso-propyl, *tert-*butyl and phenyl.

In a particular embodiment, R₁ and R₂ are each independently selected from the group consisting of phenyl, naphth-2-yl and a radical of formula Si(R₁₀)₃ wherein R₁₀ is selected from the group consisting of phenyl and 4-*tert-*butylphenyl, and wherein the phenyl group is optionally substituted with one or more groups selected from the group consisting of methyl, trifluoromethyl, isopropyl, *tert*-butyl and phenyl. More particularly, R₁ and R₂ are each 3,5-trifluoromethylphenyl.

In a preferred embodiment of the first aspect of the invention, R₁ and R₂ are the same.

In another embodiment of the first aspect of the invention, R₁ and R₂ are each independently selected from the group consisting of phenyl, naphthyl, anthracenyl, phenantrenyl, biphenylyl, phenylylnaphthalene, furyl, pyridyl, thiophenyl, pyrrolyl, indolyl, benzofuryl, benzothiofuryl, triazolyl, pyrimidyl, quinolyl, isoquinolyl, naphthyridyl and a radical of formula Si(R₁₀)₃ wherein R₁₀ is selected from the group consisting of phenyl and phenyl substituted with (C₁-C₆)alkyl, and wherein the phenyl, naphthyl, anthracenyl, phenantrenyl, biphenylyl, phenylylnaphthalene, furyl, pyridyl, thiophenyl, pyrrolyl, indolyl, indenyl, benzofuryl, benzothiofuryl, triazolyl, quinolyl, isoquinolyl, naphthyridyl and pyrimidyl groups are optionally substituted with one or more groups selected from the group consisting of nitro, (C₁-C₆)alkyl, (C₁-C₆)haloalkyl, phenyl and halogen;
R₃ and R₄ are hydrogen; and
XisO.

In a preferred embodiment of the first aspect of the invention, R₁ and R₂ are each independently selected from the group consisting of phenyl, naphthyl, anthracenyl, phenantrenyl, biphenylyl, phenylylnaphthalene, furyl, pyridyl, thiophenyl, pyrrolyl, indolyl, benzofuryl, benzothiofuryl, triazolyl, pyrimidyl, quinolyl, isoquinolyl, naphthyridyl and a radical of formula Si(R₁₀)₃ wherein R₁₀ is selected from the group consisting of phenyl and phenyl substituted with (C₁-C₆)alkyl, and wherein the phenyl, naphthyl, anthracenyl, phenantrenyl, biphenylyl, phenylylnaphthalene, furyl, pyridyl, thiophenyl, pyrrolyl, indolyl, indenyl, benzofuryl, benzothiofuryl, triazolyl, quinolyl, isoquinolyl, naphthyridyl and pyrimidyl groups are optionally substituted with one or more groups selected from the group consisting of nitro, (C₁-C₆)alkyl, (C₁-C₆)haloalkyl, phenyl and halogen;
R₃ and R₄ are hydrogen;
X is O; and
Y is -OH or -NHSO₂CF₃.

In another embodiment of the first aspect of the invention, each of the pairs R₅ and R₆, and R₇ and R₈, together with the carbon atoms to which they are attached, form a 6-membered carbocyclic ring A and a 6-membered carbocyclic ring A' respectively, said rings being independently saturated or aromatic and optionally substituted with one or more groups selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen, and the substituent of the position 6 and 6' of the fused ring system formed by the 6-membered ring A or the 6-membered ring A' and the adjacent phenyl ring in the compound of formula (I) is further selected from a radical of formula (II), wherein the radical of formula (II) is as described above.

In a further embodiment of the first aspect of the invention, the compound of formula (I) is a compound of formula (III) 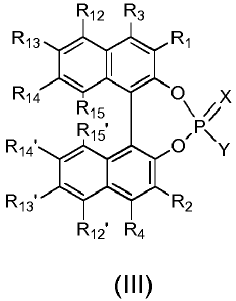 wherein R₁₂, R₁₄, R₁₅, R₁₂', R₁₄' and R₁₅' are independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen;
R₁, R₂, R₃ and R₄ are as defined in the first aspect of the invention, R₁₃ and R₁₃' are independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, halogen and a radical of formula (II) as defined above; and
with the proviso that the compound of formula (III) comprises at least one radical of formula (II).

The compound of formula (III) is the compound of formula (I) wherein each of the pairs R₅ and R₆, and R₇ and R₈, together with the carbon atoms to which they are attached, form a phenyl ring A and a phenyl ring A' respectively, said rings being optionally substituted with one or more groups selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen, and the substituent at position 6 of the fused ring system formed by the phenyl ring A or the phenyl ring A' and the adjacent phenyl ring in the compound of formula (I) is further selected from a radical of formula (II), wherein the radical of formula (II) is as described above.

In a further embodiment of the first aspect of the invention, the compounds of formula (I) are selected from the group consisting of the compounds of formula (III) as defined above
wherein R₁₂, R₁₄, R₁₅, R₁₂', R₁₄' and R₁₅' are independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen; R₁ and R₂ are each independently selected from the group consisting of phenyl, naphthyl, anthracenyl, phenantrenyl, biphenylyl, phenylylnaphthalene, furyl, pyridyl, thiophenyl, pyrrolyl, indolyl, benzofuryl, benzothiofuryl, triazolyl, pyrimidyl, quinolyl, isoquinolyl, naphthyridyl and a radical of formula Si(R₁₀)₃ wherein R₁₀ is selected from the group consisting of phenyl and phenyl substituted with (C₁-C₆)alkyl, and wherein the phenyl, naphthyl, anthracenyl, phenantrenyl, biphenylyl, phenylylnaphthalene, furyl, pyridyl, thiophenyl, pyrrolyl, indolyl, indenyl, benzofuryl, benzothiofuryl, triazolyl, quinolyl, isoquinolyl, naphthyridyl and pyrimidyl groups are optionally substituted with one or more groups selected from the group consisting of nitro, (C₁-C₆)alkyl, (C₁-C₆)haloalkyl,phenyl and halogen; R₁₃ and R₁₃' are independently selected from the group consisting of hydrogen, (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, halogen and a radical of formula (II) as defined above; and with the proviso that at least one of R₁₃ and R₁₃' is a radical of formula (II).

In a further embodiment of the first aspect of the invention, the compound of formula (I) is a compound of formula (III) as defined above;
wherein R₁₂, R₁₄, R₁₅, R₁₂', R₁₄' and R₁₅' are independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen; R₁₃ and R₁₃' are independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, halogen and a radical of formula (II) as defined above and with the proviso that one of R₁₃ and R₁₃' is a radical of formula (II).

In a further embodiment of the first aspect of the invention, the compound of formula (I) is a compound of formula (III) as defined above;
wherein R₁₂, R₁₄, R₁₅, R₁₂', R₁₄' and R₁₅' are independently selected from the group consisting of hydrogen, methyl, methoxy, trifluoromethyl, nitro, cyano, and halogen; R₁₃ and R₁₃' are independently selected from the group consisting of hydrogen, (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, halogen and a radical of formula (II) as defined above and with the proviso that one of R₁₃ and R₁₃' is a radical of formula (II).

In an even further embodiment of the first aspect of the invention, the compound of formula (I) is a compound of formula (III) as defined above; wherein R₁₂, R₁₄, R₁₅, R₁₂', R₁₄' and R₁₅' are hydrogen; R₁₃ and R₁₃' are independently selected from the group consisting of hydrogen, (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, halogen and a radical of formula (II) as defined above and with the proviso that one of R₁₃ and R₁₃' is a radical of formula (II).

In an even further embodiment of the first aspect of the invention, the compound of formula (I) is a compound of formula (III) as defined above; wherein R₁ and R₂ are the same; R₁₂, R₁₄, R₁₅, R₁₂', R₁₄' and R₁₅' are hydrogen; R₁₃ and R₁₃' are independently selected from the group consisting of hydrogen, (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, halogen and a radical of formula (II) as defined above and with the proviso that one of R₁₃ and R₁₃' is a radical of formula (II).

In another embodiment of the first aspect of the invention, in the radical of formula (II), Z is a biradical linked to B and L selected from the group consisting of -O-, -S-, -NR₁₁-, -NR₁₁SO₂- and -SO₂NR₁₁-, wherein R₁₁ is as defined in the first aspect of the invention. Preferably, in the radical of formula (II), Z is a biradical linked to B and L selected from the group consisting of -O-and -SO₂NR₁₁-, wherein R₁₁ is as defined in the first aspect of the invention. Even more preferably, in the radical of formula (II), Z is -O-.

In a further embodiment of the first aspect of the invention, in the radical of formula (II), B and L are each a biradical independently selected from the group consisting of methylene -CH₂-, ethylene -CH₂-CH₂-, phenylene -C₆H₄-, benzylene -CH₂-C₆H₄-, and -CH₂-C₆H₄-CH₂-. In an even further embodiment, B and L are each a biradical independently selected from the group consisting of methylene -CH₂-, phenylene -C₆H₄-, benzylene -CH₂-C₆H₄-, and -CH₂-C₆H₄-CH₂-. In an even further embodiment, B and L are each a biradical independently selected from the group consisting of methylene -CH₂-, phenylene -C₆H₄- and benzylene -CH₂-C₆H₄-. In an even further embodiment, B and L are each a methylene biradical.

In a further embodiment of the first aspect of the invention, in the radical of formula (II), L and B are independently selected from the group consisting of the group consisting of methylene, phenylene and benzylene; and Z is selected from the group consisting of -O- and -NSO₂R₁₁-, wherein R₁₁ is as defined in the first aspect of the invention.

In a particular embodiment of the first aspect of the invention, in the radical of formula (II), n is 1.

In another particular embodiment of the first aspect of the invention, in the radical of formula (II), Pol is a polymeric support comprising a polymer selected from the group consisting of polystyrene, a polyacrylate, a polyacrylamide, a polyvinylpyrrolidinone, a polysiloxane, a polybutadiene, a polyisoprene, a polyalkane, a polyoxazoline, a polyether, mixtures and copolymers thereof. Particularly, Pol is polystyrene, and more particularly Pol is a cross-linked polystyrene-divinylbenzene polymer. In this particular embodiment, the polymeric support is a cross-linked polystyrene-divinylbenzene polymer wherein the polymer comprises from 0.5 to 2 weight% of divinylbenzene. Such polymers are porous and are known in the art with the commercial name of Merrifield resin.

In a particular embodiment of the first aspect of the invention, the compound of formula (I) comprises from 0.01 to 5 millimoles of the moiety of formula-O₂PXY per gram of the compound of formula (I). More particularly, the compound of formula (I) comprises from 0.05 to 2 millimoles of the moiety of formula O₂PXY per gram of the compound of formula (I). Even more particularly, the compound of formula (I) comprises from 0.1 to 1 millimole of the moiety of formula O₂PXY per gram of the compound of formula (I).

In a particular embodiment of the first aspect of the invention, the compound of formula (I) is selected from the group consisting of the compounds of formula (IIIa), (IIIb), (IIIc), (IIId), (IIIe), (IIIf), (IIIg), (IIIh) and (IIIi) 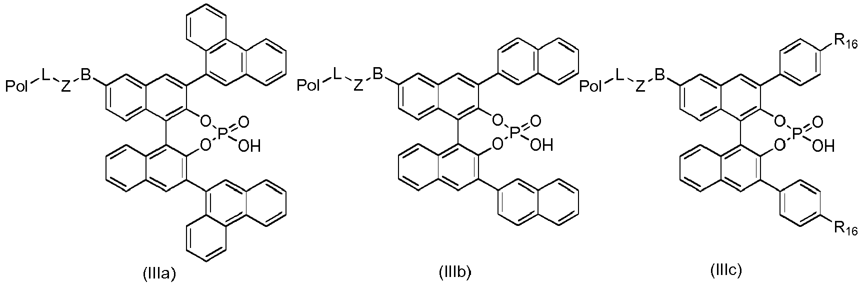 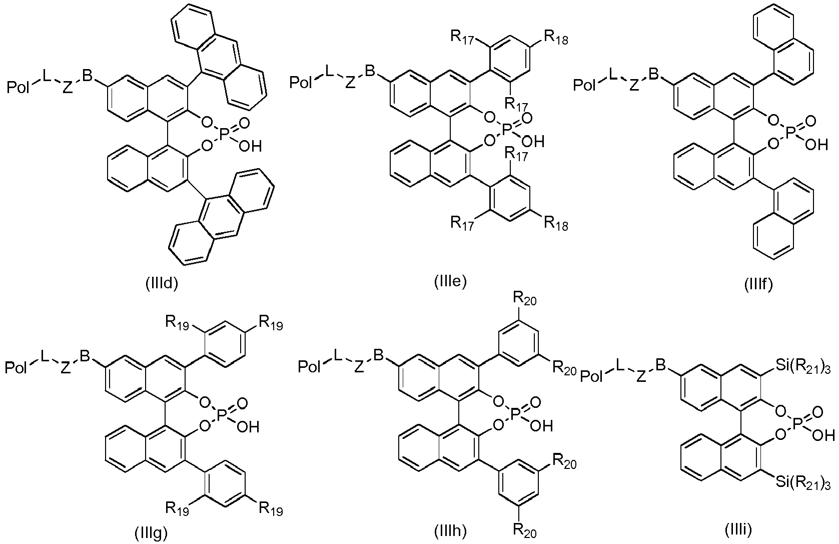 wherein
R₁₆ is a radical selected from the group consisting of hydrogen, nitro, chloro, naphth-2-yl, phenyl, 3,5-(ditrifluoromethyl)phenyl, tolyl, *tert*-butyl, trifluoromethyl and isopropyl;
R₁₇ and R₁₈ are each independently selected from the group consisting of hydrogen, methyl, isopropyl, *tert*-butyl, and trifluoromethyl;
R₁₉ and R₂₀ are each trifluoromethyl or phenyl; and
R₂₁ is selected from the group consisting of phenyl and *tert*-butylphenyl.

In a more particular embodiment of the first aspect of the invention, the compound of formula (I) is selected from the group consisting of the compounds of formula (IIIa), (IIIb), (IIIc), (IIId), (IIIe), (IIIf), (IIIg), (IIIh) and (IIIi), wherein Z is O.

In a more particular embodiment of the first aspect of the invention, the compound of formula (I) is selected from the group consisting of the compounds of formula (IIIa), (IIIb), (IIIc), (IIId), (IIIe), (IIIf), (IIIg), (IIIh) and (IIIi), wherein Z is O and B and L are methylene.

In a more particular embodiment of the first aspect of the invention, the compound of formula (I) is selected from the group consisting of the compounds of formula (IIIa), (IIIb), (IIIc), (IIId), (IIIe), (IIIf), (IIIg), (IIIh) and (IIIi), wherein Z is O; B and L are methylene and Pol is a polystyrene-divinylbenzene cross-linked polymer.

In another particular embodiment, the compound of formula (I) is a compound of formula (IIIh) as defined above wherein R₂₀ is trifluoromethyl.

More particularly, the compound of formula (I) is a compound of formula (IIIh) as defined above, wherein Z is -O-; B and L are methylene, and wherein R₂₀ is trifluoromethyl.

Even more particularly, the compound of formula (I) is a compound of formula (IIIh) as defined above, wherein Z is -O-; B and L are methylene, R₂₀ is trifluoromethyl; and Pol is a polystyrene-divinylbenzene cross-linked polymer.

The beneficial contribution of the catalysts of the invention in the preparation of a 3-indolylmethanamine is an enhanced reactivity (higher turnover numbers can be achieved) in the presence of a lower excess amount of reagents (e.g. indole derivative), along with an improved enantioselectivity with respect to the catalysts of the prior art.

According to the second aspect, the invention provides a process for the preparation of the catalysts of the invention.

In preferred embodiments of the second aspect, in the compounds of formula (IV) used in step (i), R₃ and R₄ are as defined in any of the embodiments of the first aspect of the invention.

In further preferred embodiments of the second aspect of the invention, PG is selected from the group consisting of benzyl, a radical of formula Si[(C₁-C₆)alkyl]₃ and methoxymethyl. More preferably, PG is methoxymethyl.

In a preferred embodiment of the second aspect of the invention, in the compound of formula (IV) used in step (i), R₂₂ and R₂₃ are each independently selected from the group consisting of (C₆-C₂₀ aryl); (C₅-C₂₀)heteroaryl; a radical of formula Si(R₁₀)₃ wherein R₁₀ is selected from the group consisting of phenyl and phenyl substituted with a group selected from the group consisting of nitro, halogen, (C₁-C₆)haloalkyl and (C₁-C₆)alkyl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, phenyl, and halo.

In another embodiment of the second aspect of the invention, in the compound of formula (IV) used in step (i) R₂₂ and R₂₃ are each independently selected from the group consisting of phenyl, naphthyl, anthracenyl, phenantrenyl and a radical of formula Si(R₁₀)₃ wherein R₁₀ is selected from the group consisting of phenyl and phenyl substituted with (C₁-C₆)alkyl, and wherein the phenyl, naphthyl, anthracenyl, phenantrenyl groups are optionally substituted with one or more groups selected from the group consisting of nitro, halogen, (C₁-C₆)alkyl, (C₁-C₆)haloalkyl and phenyl.

In another embodiment, R₂₂ and R₂₃ are each independently selected from the group consisting of phenyl, naphth-2-yl, anthracen-9-yl, phenantren-9-yl and a radical of formula Si(R₁₀)₃ wherein R₁₀ is selected from the group consisting of phenyl and 4-tert-butylphenyl, and wherein the phenyl group is optionally substituted with one or more groups selected from the group consisting of methyl, trifluoromethyl, iso-propyl, *tert*-butyl and phenyl.

In a particular embodiment, R₂₂ and R₂₃ are each independently selected from the group consisting of phenyl, naphth-2-yl and a radical of formula Si(R₁₀)₃ wherein R₁₀ is selected from the group consisting of phenyl and 4-*tert-*butylphenyl, and wherein the phenyl group is optionally substituted with one or more groups selected from the group consisting of methyl, trifluoromethyl, iso-propyl, *tert*-butyl and phenyl. More particularly, R₂₂ and R₂₃ are each 3,5-trifluoromethylphenyl.

In a preferred embodiment of the second aspect of the invention, R₂₂ and R₂₃ are the same.

In another embodiment of the second aspect of the invention, in the compound of formula (IV) used in step (i), each of the pairs R₂₄ and R₂₅, and R₂₆ and R₂₇, together with the carbon atoms to which they are attached, form a 6-membered carbocyclic ring D and 6-membered carbocyclic ring D' respectively, said rings being independently saturated or aromatic and optionally substituted with one or more groups selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen, and the substituent at position 6 of the fused ring system formed by the 6-membered ring D or the 6-membered ring D' and the adjacent phenyl ring in the compound of formula (IV) is further selected from a radical of formula (II') as defined in the second aspect of the invention.

In a further embodiment of the second aspect of the invention, the compound of formula (IV) used in step (i) is a compound of formula (VI) 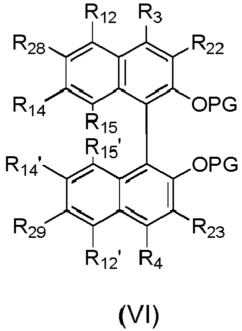 wherein R₃, R₄, R₁₂, R₁₄, R₁₅, R₁₂', R₁₄' and R₁₅' are as defined in any embodiment of the first aspect of the invention;
R₂₂ and R₂₃ are as defined above; and
R₂₈ and R₂₉ are independently selected from the group consisting of hydrogen, (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, halogen and a radical of formula (II') as defined in the second aspect of the invention; and with the proviso that the compound of formula (VI) comprises at least one radical of formula (II').

The compound of formula (VI) is a compound of formula (IV) wherein each of the pairs R₂₄ and R₂₅, and R₂₆ and R₂₇, together with the carbon atoms to which they are attached, form a phenyl ring D and phenyl ring D' respectively, said rings being optionally substituted with one or more groups selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen, and the substituent at position 6 of the fused ring system formed by the phenyl ring D or the phenyl ring D' and the adjacent phenyl ring in the compound of formula (IV) is further selected from a radical of formula (II') as defined in the second aspect of the invention.

In an even further embodiment of the second aspect of the invention, the compound of formula (IV) used in step (i) is a compound of formula (VI) as defined above; wherein R₂₂ and R₂₃ are the same; R₁₂, R₁₄, R₁₅, R₁₂', R₁₄' and R₁₅' are hydrogen; R₂₈ and R₂₉ are independently selected from the group consisting of hydrogen, (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, halogen and a radical of formula (II') as defined above and with the proviso that one of R₂₈ and R₂₉ is a radical of formula (II').

In a preferred embodiment of the second aspect of the invention, in the radical of formula (II'), FG is selected from the group consisting of the radicals of formula -OH, -SH, -NHR₁₁, -COOH, -COCl and -SO₂Cl; wherein R₁₁ is selected from the group consisting of H, (C₁-C₆)alkyl, phenyl and benzyl.

In a further embodiment of the second aspect of the invention, the polymeric support comprises at least a terminal group selected from the group consisting of halogen, -NHR₁₁, -COOH, -COCl and -SO₂Cl; wherein R₁₁ is selected from the group consisting of H, (C₁-C₆)alkyl, phenyl and benzyl.

According to the second aspect of the invention, when, in the compound of formula (I), Z is -O-, then the polymeric support used in step (i) comprises at least a terminal halogen group; and, in the compound of formula (IV), FG is hydroxyl.

Similarly, when, in the compound of formula (I), Z is -S-, then the polymeric support used in step (i) comprises at least a terminal halogen group; and, in the compound of formula (IV), FG is -SH.

Similarly, when, in the compound of formula (I), Z is -CONR₁₁-, then the polymeric support used in step (i) comprises at least a terminal group selected from the group consisting of -COOH or -COCl; and, in the compound of formula (IV), FG is -HNR₁₁.

Similarly, when in the compound of formula (I), Z is -NR₁₁CO-, then the polymeric support used in step (i) comprises at least a terminal group of formula -HNR₁₁; and, in the compound of formula (IV), FG is selected from the group consisting of -COOH or -COCl.

Similarly, when, in the compound of formula (I), Z is -SO₂NR₁₁-, then the polymeric support used in step (i) comprises at least a terminal group of formula -SO₂Cl; and, in the compound of formula (IV), FG is -HNR₁₁.

Similarly, when, in the compound of formula (I), Z is -NR₁₁SO₂-, then the polymeric support used in step (i) comprises at least a terminal group of formula -HNR₁₁; and, in the compound of formula (IV), FG is of formula - SO₂Cl.

Similarly, when, in the compound of formula (I), Z is -NR₁₁-, then the polymeric support used in step (i) comprises at least a terminal halogen group; and, in the compound of formula (IV), FG is -HNR₁₁; or, alternatively, the polymeric support used in step (i) comprises at least a terminal group of formula -HNR₁₁; and, in the compound of formula (IV), FG is halo.

In most preferred embodiments, the polymeric support comprises at least a halogen group; and, in the compound of formula (IV), FG is hydroxyl. More preferably, the halogen group is selected from the group consisting of chloride, bromide and iodide.

According to the second aspect of the invention, step (ii) of the preparation of the compound of formula (I) comprises unprotecting the alcohols in the product obtained in (i). Suitable methods to carry out this deprotection step are well known in the art and will become apparent to the skilled in the art. For instance, when PG is benzyl, a suitable deprotection method of the alcohols is catalytic hydrogenation, preferably in the presence of a palladium catalyst. Alternatively, when PG is benzoyl, a suitable deprotection method of the alcohols comprises treating the compound obtained in (i) with water, preferably in basic medium. Alternatively, when PG is a radical of formula Si[(C₁-C₆)alkyl]₃ as described above, a suitable deprotection method comprises treating the compound obtained in (i) with fluoride anions or in acidic medium. Alternatively, when OPG is an ether, such as methoxy (when PG is methyl) or methoxymethyl (when PG is methoxymethyl), a suitable deprotection method comprises treating the compound obtained in (i) in acidic medium.

According to the second aspect of the invention, step (iii) of the preparation of the compound of formula (I) comprises reacting the compound obtained in step (ii) with phosphorus oxychloride or thiophosphoryl chloride. When, in the compound of formula (I), X is O, then step (iii) of the preparation of the compound of formula (I) comprises reacting the compound obtained in step (ii) with phosphorus oxychloride. When, in the compound of formula (I), X is S, then step (iii) of the preparation of the compound of formula (I) comprises reacting the compound obtained in step (ii) with thiophosphoryl chloride.

Step (iv) of the preparation of the compound of formula (I) comprises reacting the compound obtained in step (iii) with water or ammonia. When, in the compound of formula (I), Y is OH, then step (iv) of the preparation of the compound of formula (I) comprises reacting the compound obtained in step (iii) with water. When, in the compound of formula (I), Y is a radical of formula -NSO₂R₉, then step (iv) of the preparation of the compound of formula (I) comprises reacting the compound obtained in step (iii) with ammonia.

Optionally, when, in the compound of formula (I), Y is a radical of formula - NSO₂R₉, then the preparation of the compound of formula (I) further comprises reacting the compound obtained in step (iv) with a compound of formula R₉SO₂Cl.

The inventors have found that the catalysts of the invention are useful in the preparation of 3-indolylmethanamine derivatives by Friedel-Crafts condensation of indole derivatives onto imines. In particular, the resulting performance, ease of recovery and robustness of the catalysts of the invention makes them suitable for the preparation of 3-indolylmethanamines.

Thus, in a preferred embodiment of the third aspect, the compound of formula (I) is used as a catalyst in the preparation of a 3-indolylmethanamine.

In a particular embodiment of the fourth aspect of the invention, the process for the preparation of a 3-indolylmethanamine is a continuous flow process.

Being non-soluble in the reaction solvent, the compounds of the invention can be incorporated into a reactor, preferably a tubular reactor or a column, through which a solution of the reagents is pumped in, thereby allowing the catalysed reaction to occur and high turnover numbers to be reached. This is advantageous as it allows for the highly productive, continuous and non-stopping preparation of the products in high yields and enantioselectivity. Such column or reactor filled with the compound of the invention is also part of the invention.

Additionally, this allows for the high-yielding and selective preparation of a large number of compounds in a reduced amount of time, by carrying out the following steps in a sequential manner:
(i) inject the solution(s) of reagents into the reactor;
(ii) collect the product at the outlet of the reactor;
(iii) wash the reactor with a solvent; and
(iv) carry out steps (i), (ii) and (iii) with different reagents than the reagents used in (i).

In a particular embodiment, the fourth aspect of the invention relates to a process for the preparation of a 3-indolylmethanamine of formula (XI) wherein, in the compound of formula (X), R₃₀ is selected from hydrogen, (C₁-C₆)alkyl, and benzyl. More particularly, R₃₀ is hydrogen.

In another particular embodiment, the fourth aspect of the invention relates to a process for the preparation of a 3-indolylmethanamine of formula (XI) wherein, in the compound of formula (X), R₃₁, R₃₂, R₃₃, R₃₄ and R₃₅ are selected from the group consisting of hydrogen, (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, and halogen. More particularly, R₃₁, R₃₂, and R₃₅ are hydrogen.

In another particular embodiment, the fourth aspect of the invention relates to a process for the preparation of a 3-indolylmethanamine of formula (XI) wherein, in the compound of formula (IX), R₃₇ is selected from the group consisting of (C₁-C₆)alkyl; (C₁-C₆)haloalkyl; benzyl; fluorenyl; phenyl; pyridyl; phenyl substituted with one or more group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen; and pyridyl substituted with one or more group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen. More particularly, R₃₇ is selected from the group consisting of (C₁-C₆)alkyl, phenyl, tolyl, benzyl, fluorenyl and pyridyl.

In another particular embodiment, the fourth aspect of the invention relates to a process for the preparation of a 3-indolylmethanamine of formula (XI) wherein, in the compound of formula (IX), R₃₆ is selected from the group consisting of (C₆-C₂₀ aryl); (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NS0₂R₃₇ and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NS0₂R₃₇ and halogen; and R₃₇ is as defined above.

In preferred embodiments of the fourth aspect of the invention, the invention relates to a process for the preparation of a 3-indolylmethanamine of formula (V), which comprises contacting a compound of formula (VII) with a compound of formula (VIII) in the presence of a compound of formula (I); 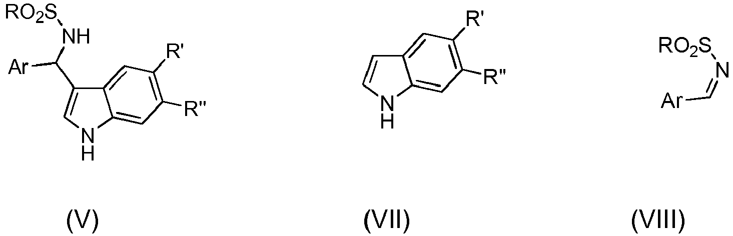 wherein:
Ar is selected from the group consisting of (C₆-C₂₀ aryl); (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R and halogen;
R is selected from the group consisting of phenyl, tolyl, fluorenyl, 2-pyridyl, benzyl, and *tert*-butyl; and
R' and R" are independently selected from the group consisting of hydrogen, (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, and halogen.

The compound of formula (VII) is a compound of formula (X) wherein R₃₀, R₃₁, R₃₂ and R₃₅ are hydrogen; and R₃₃ and R₃₄ are independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen.

The compound of formula (VIII) is a compound of formula (IX) wherein:
R₃₆ is selected from the group consisting of (C₆-C₂₀ aryl); (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R and halogen; and
R₃₇ is selected from the group consisting of phenyl, tolyl, fluorenyl, 2-pyridyl, benzyl, and *tert*-butyl.

The compound of formula (V) is a compound of formula (XI) wherein:
R₃₀, R₃₁, R₃₂ and R₃₅ are hydrogen; and R₃₃ and R₃₄ are independently selected from the group consisting of hydrogen, (C₁-C₆)alkyl, (C₁-C₆)alkyloxy, (C₁-C₆)haloalkyl,nitro, cyano, (C₁-C₆)alkylcarbonyl, (C₁-C₆)alkylcarbonyloxy, (C₁-C₆)alkyloxycarbonyl, and halogen; R₃₆ is selected from the group consisting of (C₆-C₂₀ aryl); (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NS0₂R₃₇ and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NS0₂R₃₇ and halogen; and
R₃₇ is selected from the group consisting of phenyl, tolyl, fluorenyl, 2-pyridyl, benzyl, and *tert*-butyl.

In more preferred embodiments of the fourth aspect of the invention, in the compound of formula (V), Ar is selected from the group consisting of phenyl, 4-chlorophenyl, 4-bromophenyl, 4-tolyl, 3-tolyl, 2-tolyl, 4-methoxyphenyl, 2-tosylformimidatophenyl and 4-nitrophenyl.

In more preferred embodiments of the fourth aspect of the invention, in the compound of formula (V), R is selected from the group consisting of phenyl, tolyl, and 2-pyridyl.

In more preferred embodiments of the fourth aspect of the invention, in the compound of formula (V), R' and R" are each independently selected from the group consisting of hydrogen, methoxy, methyl, bromo and chloro.

In a preferred embodiment of the fourth aspect of the invention, the molar ratio of the compound of formula (X) to the compound of formula (IX) is comprised from 1:1 to 3:1; more preferably from 1:1 to 2:1.

Using the catalysts described in the state of the art, a 3-indolylmethanamine of formula (XI) can be prepared by reacting a compound of formula (IX) with a compound of formula (X) using a molar ratio of the compound of formula (X) to the compound of formula (IX) of 5:1. Thus, the catalysts of the invention allow decreasing the amount of excess compound of formula (X) in the preparation of a 3-indolylmethanamine without observing the formation of undesired side products.

In preferred embodiments, the preparation of a 3-indolylmethanamine of formula (XI) described above comprises contacting a compound of formula (X) with a compound of formula (IX) in the presence of the compound of formula (I) at room temperature, preferably at a temperature comprised from 10 °C to 30°C.

Even when used at room temperature or at a temperature comprised from 10 °C to 30 °C, in the preparation of 3-indolylmethanamines of formula (XI), the catalysts of the invention provide high yields and enantioselectivity.

In a more preferred embodiment, the fourth aspect of the invention relates to a process for the preparation of a 3-indolylmethanamine of formula (XI) wherein the molar ratio of the compound of formula (X) to the compound of formula (IX) is comprised from 1:1 to 3:1; and the process is carried out at a temperature comprised from 10 °C to 30 °C.

In preferred embodiments, the preparation of a 3-indolylmethanamine of formula (XI) described above comprises contacting a compound of formula (X) with a compound of formula (IX) in the presence of the compound of formula (I), wherein the amount of the compound of formula (I) is such that the amount of the moiety of formula Y in the compound of formula (I) is comprised from 0.5 to 10 moles per each 100 moles of compounds of formula (IX).

It is advantageous since the catalysts of the invention can be used in minor amounts than the ones described in the state of the art.

In preferred embodiments, the preparation of a 3-indolylmethanamine of formula (XI) described above comprises contacting a compound of formula (X) with a compound of formula (IX) in the presence of a polar aprotic solvent. In the context of the invention, a polar aprotic solvent is polar solvent lacking of acidic hydrogen atoms able to form hydrogen bonds with the solute. Preferably, the solvent is selected from the group consisting of dichloromethane, dichloroethane, chloroform, toluene, N,N-dimethylformamide, tetrahydrofuran, and 1,4-dioxane.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### General information

Unless otherwise stated, all commercial reagents were used as received and all reactions were carried out directly under open air. Merrifield resin (1% PS-DVB, f = 0.53 mmol of Cl per gram of resin) was purchased from Novabiochem. In each case the extent of the supporting process and the functionalization of the final resin was determined by elemental analysis. All flash chromatography was carried out using 60 mesh silica gel and dry-packed columns. NMR spectra were recorded in a Bruker Advance 400 Ultrashield NMR spectrometer for ¹H NMR at 400 or 500 MHz, and ¹³C at 100 or 126 MHz. The solvent employed in each case is indicated between brackets. Chemical shifts (δ) in ppm are indicated with respect to the reference used: tetramethylsilane (TMS, internal reference) in ¹H NMR, solvent residual peak for ¹³C NMR spectra. ATR measurements were carried out in a FT-IR Alpha spectrometer with DTGS detector, using a Bruker ATR accessory with diamond crystal. Specific rotation was determined by using a Jasco P-1030 polarimeter equipped with a sodium lamp, and a photomultiplier tube detector with a 589 nm filter, in a polarimetry cell of 100 mm length. Concentration in g/100 ml and solvent used are indicated in brackets. High resolution mass spectrometry analyses were performed in a Waters LCD PremierTM instrument operating in ESI (Electro-Spray Ionization) mode or APCI (Atmospheric-Pressure Chemical Ionization) mode. Elemental analyses were performed by MEDAC Ltd. (Surrey, UK) on a LECO CHNS 932 micro-analyzer. High performance liquid chromatography (HPLC) was performed on Agilent Technologies chromatograph (Serie1200), using Chiralcel OD-H or Chiralpak IA column and guard column. The continuous flow experiments were carried out using a Syrris Asia pump with pressurized input and backpressure regulator. The packed-bed reactor was a ¼ inch Teflon tube of 17 cm length. Conversion was monitored by real time IR spectroscopy thanks to the Mettler Toledo FlowIR.

### Example 1: Preparation of polymer supported catalyst (IIIh)

### Step 1: Preparation of (R)-4-((2,2'-bis(methoxymethoxy)-[1,1'-binaphthalen]-6-yl)methoxy)(tert-butyl)dimethylsilane 2

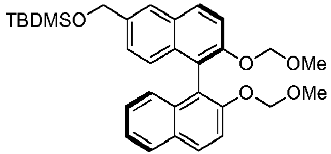

Under argon atmosphere, *tert*-butyldimethylchlorosilane (2.82 g, 18.16 mmol) was added to a mixture of (2,2'-bis(methoxymethoxy)-[1,1'-binaphthalen]-6-yl)methanol **1** (6.12g, 15.13 mmol, 1 was prepared as described in Tetrahedron: Asymmetry 2001, 12, 2589.) and imidazole (2.58 g, 37.80 mmol) in dry DMF (23 mL) at room temperature. After being stirred for 2 h, water (100 mL) was added to quench the reaction. The mixture was then extracted with ethyl acetate (3 x 50 mL). The combined organic extracts were washed with brine (100 mL), dried over MgSO₄, filtered and evaporated to give the crude product as a colorless oil. It was purified by flash chromatography on silica gel to afford the final product in quantitative yield (7.90 g, 15.13 mmol).
**[α]²⁷_{D}:** +5.1° (*c* 1.00, CH₂Cl₂).
**IR** (ATR): 2954, 2927, 2898, 2855, 1593, 1505, 1482 cm⁻¹.
**HRMS** (ESI+): *m*/z calculated for C₃₁H₃₈NaO₅Si [M+Na]⁺: 541.2381, found 541.2399.

### Step 2: Preparation of (R)-4-tert-butyl((3,3'-dibromo-2,2'-bis(methoxymethoxy)-[1,1'-binaphthalen]-6-yl)methoxy) dimethylsilane 3

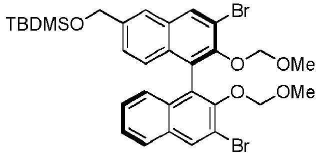

To a solution of compound **2** (1.09 g, 2.11 mmol) in anhydrous THF (8.5 mL), n-BuLi (3.16 mL, 5.06 mmol) was added dropwise at -78 °C under Ar atmosphere. The mixture was allowed to warm to 0 °C, stirred for 1 h and then cooled to -78 °C again. Then, a solution of bromine (0.33 mL, 6.32 mmol) was added dropwise and the mixture was stirred overnight at room temperature. Then, it was poured into a solution of saturated Na₂SO₃ (20 mL), the organic layer was separated and the aqueous phase was extracted with EtOAc (3 x 10 mL). The combined organic extracts were washed with brine and dried over Na₂SO₄. The mixture was filtered and evaporated under reduced pressure. The resulting crude was purified by column chromatography on silica gel (Hexane/EtOAc 90:10) to give a light brown oil in 70% yield (1.00 g, 1.48 mmol).
**[α]²⁷_{D}:** +1.7° (*c* 3.15, CH₂Cl₂).
**IR** (ATR): 3062, 2953, 2928, 2884, 1578, 1563, 1465, 1386, 1350 cm⁻¹.
**HRMS** (APCI+): *m*/*z* calculated for C₃₁H₃₆NaBr₂O₅Si [M+Na]⁺: 697.0591, found 697.0598.

### Step 3: Preparation of (R)-4-((3,3'-bis(3,5-bis(trifluoromethyl)phenyl)-2,2'-bis(methoxymethoxy)-[1,1'-binaphthalen]-6-yl)methoxy)(tert-butyl)dimethylsilane 4

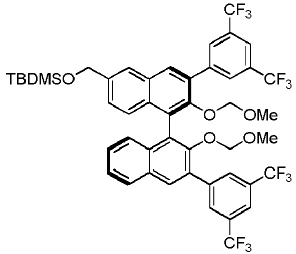

The bromo derivative **3** (3.97 g, 5.87 mmol), (3,5-bis(trifluoromethyl)phenyl)boronic acid (3.78 g, 14.67 mmol), Pd₂(dba)₃ (0.16 g, 0.18 mmol), Sphos (0.29 g, 0.70 mmol) and K₃PO₄ (6.23 g, 29.3 mmol) were dissolved in dry toluene (12 mL) in a Schlenk flask under argon atmosphere. The reaction mixture was stirred overnight at 110 °C. Then, it was allowed to cool down to room temperature and it was filtered through a plug of celite. The filtrate was evaporated under reduced pressure to give a residue. Subsequent purification by column chromatography on silica gel (from *c*-Hexane to *c*-Hexane/EtOAc 90:10) afforded the product in 86% yield.
**[α]²⁷_{D}:** -6.0° (*c* 1.10, CH₂Cl₂).
**IR** (ATR): 2957, 2930, 2858, 1471, 1378, 1278, 1129 cm⁻¹.
**HRMS** (ESI+): m/z calculated for C₄₇H₄₂F₁₂NaO₅Si [M+Na]⁺: 965.2502, found 965.2496.
**m.p.:** 254-274 °C (dec.).

### Step 4: Preparation of (R)-4-(3,3'-bis(3,5-bis(trifluoromethyl)phenyl)-2,2'-bis(methoxymethoxy)-[1,1'-binaphthalen]-6-yl)methanol 5

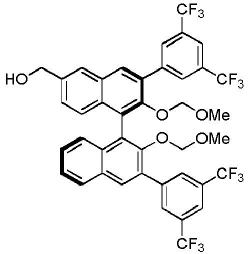

Compound **4** (4.70 g, 4.98 mmol) was dissolved in dry THF (28.5 mL) under argon atmosphere, a 1 M solution of Bu₄NF in THF (9.97 mL, 9.97 mmol) was added at 0 °C and the reaction was stirred at room temperature for 4 h. Then, water (10 mL) was added to quench the reaction, the organic layer was separated and the aqueous phase was extracted with EtOAc (3 x 10 mL). The combined organic extracts were washed with brine and dried over Na₂SO₄. The mixture was filtered and evaporated under reduced pressure. The resulting crude was purified by column chromatography on silica gel (from Hex:EtOAc 90:10 to Hex:EtOAc 70:30) to afford the product in 89% yield (3.63 g, 4.38 mmol).
**[α]²⁷_{D}:** -7.0° (*c* 1.00, CH₂Cl₂).
**IR** (ATR): 1366, 1276, 1278,1123 cm⁻¹.
**HRMS** (ESI+): m/z calculated for C₄₁H₂₈NaF₁₂O₅ [M+Na]⁺: 851.1637, found 851.1669.
**m.p.:** 96-100 °C.

### Step 5 (step (i)): Preparation of (R)-4-(3,3'-bis(3,5-bis(trifluoromethyl)phenyl)-2,2'-bis(methoxymethyloxy)-[1,1'-binaphthalen]-6-yl)methoxymethyl polystyrene cross-linked with divinylbenzene at 1% (Merrifield resin) 6

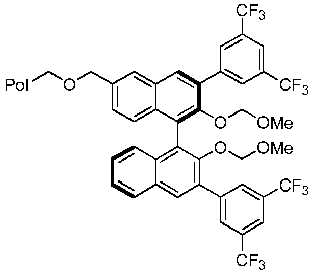

To a suspension of NaH (0.165 g, 1.15 mmol) in dry DMF (16 mL) was added compound 5 (dissolved in 10 mL of DMF) at room temperature, under argon atmosphere. In a second flask, the polystyrene cross-linked with divinylbenzene and functionalized with chlorine (Merrifield resin, 1% DVB, 2.16 g, 1.08 mmol of active chlorine) and Bu₄NI (0.050 g, 0.14 mmol) were suspended in dry DMF (13 mL) in order to swell the resin. Both suspensions were shaken for 1 h and the first one was then added via cannula to the resin. The reaction mixture was shaken at this temperature until Raman spectroscopy showed complete disappearance of the chlorine band (678 cm⁻¹). The resulting resin was filtered and washed successively with water (200 mL), water/THF 1:1 (200 mL), THF (200 mL), THF/CH₂Cl₂ 1:1 (200 mL) and CH₂Cl₂ (200 mL). Then it was allowed to dry overnight under vacuum at 50 °C and 2.66 g of **6** were isolated.
**F elemental analysis** (%): 9.08 (average of two measurements)
**f:** 0.39 (quantitative anchoring, ***f**ₘₐₓ*: 0.36 mmolₘₒₙₒₘₑᵣ/gᵣₑₛᵢₙ)

### Step 6 (step (ii)): Preparation of (R)-4-(3,3'-bis(3,5-bis(trifluoromethyl)phenyl)-2,2'-dihydroxy-1,1'-binaphthalen-6-yl)methoxymethyl polystyrene cross-linked with divinylbenzene at 1% (Merrifield resin) 7

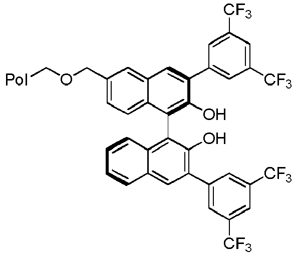

A mixture of compound 6 (2.66 g, 0.96 mmol) and HCl in EtOAc (2 M, 23 mL, 46 mmol) was shaken at room temperature overnight. After filtration, the resin was washed successively with EtOAc (200 mL), EtOAc/CH₂Cl₂ (200 mL) and CH₂Cl₂ (200 mL) and dried overnight under vacuum at 50 °C to afford 7 (2.50 g).

### Step 7 (step (iii) and (iv)): Preparation of (R)-4-(3,3'-bis(3,5-bis(trifluoromethyl)phenyl)-2,2'-diyl hydrogen phosphate-1,1'-binaphthalen-6-yl)methoxymethyl polystyrene cross-linked with divinylbenzene at 1% (Merrifield resin) (IIIh)a

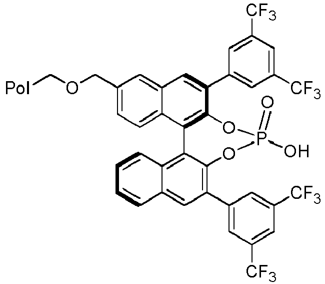

Compound 7 (2.50 g, 0.82 mmol) was suspended in dry pyridine (14 mL) under argon atmosphere and POCl₃ (0.15 mL, 1.57 mmol) was added at room temperature. The reaction mixture was shaken for 5 h. Then, 5 mL of water were added and the resulting suspension was shaken for additional 30 min. After filtration, the resin was washed successively with 1 N aqueous HCl (300 mL), water (300 mL), THF (200 mL), stirred for 10 minutes in 2 M HCl in EtOAc, washed with 200 mL of EtOAc and CH₂Cl₂ (200 mL) and dried overnight under vacuum at 50 °C to afford 2.35 g of catalyst **IIIha.**
**F elemental analysis** (%): 6.00 (average of 4 measurements)
**f:** 0.26 mmolₘₒₙₒₘₑᵣ/gᵣₑₛᵢₙ (70% of functionalization, ***fmax***: 0.37 mmolₘₒₙₒₘₑᵣ/gᵣₑₛᵢₙ)

### Example 2: Preparation of 3-indolylmethanamine compounds of formula (V) in batch conditions

### Examples 2a-2g: Preparation of (S)-N-((1H-indol-3-yl)(phenyl)methyl-4-methylbenzenesulfonamide

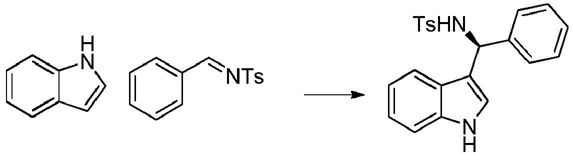

General procedure: Indole (amount indicated in Table 1), *N*-benzylidene-4-methylbenzenesulfonamide (0.07 mmol, 1 equivalent) and the compound of formula **(IIIh)a** obtained in Example 1 (loading indicated in Table 1) were placed in a vial and the solvent was added (0.4 mL when the concentration was 0.16 M, or, alternatively, 0.8 mL when the concentration was 0.08 M) was added. The reaction mixture was shaken at room temperature until Thin Layer Chromatography monitoring showed complete consumption of the starting compound of formula (VIII) and for the time indicated in Table 1. Then, the resin was filtered and the filtrate directly purified by column chromatography on silica gel (from CH₂Cl₂ to CH₂Cl₂/EtOAc 96:4) to yield the product as a white solid.

Table 1 shows the results obtained.

**Table 1**

| Example | Solvent | Concentration of compound (VIII) (M) | **(IIIh)a** Cat. loading (mol%) | Time (h) | Indole equiv. | Yield (%) | ee (%) |
|---|---|---|---|---|---|---|---|
| 2a | Toluene | 0.08 | 8 | 2.5 | 1.3 | 96 | 85 |
| 2b | CH₂Cl₂ | 0.08 | 8 | 21 | 1.3 | 63 | 97 |
| 2c | CH₂Cl₂ | 0.08 | 16 | 21 | 1.3 | 63 | 97 |
| 2d | CH₂Cl₂ | 0.08 | 10 | 5 | 2 | 92 | 92 |
| 2e | CH₂Cl₂ | 0.08 | 10 | 5 | 3 | 95 | 91 |
| 2f | CH₂Cl₂ | 0.08 | 10 | 6 | 1.5 | 77 | 94 |
| 2g | CH₂Cl₂ | 0.16 | 10 | 2.5 | 1.5 | 81 | 93 |

As a comparative example, Kang and co-workers reported that the same reaction, when carried out in the presence of 10 moles of compound of formula (I') per 100 moles of *N*-benzylidene-4-methylbenzenesulfonamide, and using toluene as a solvent, and wherein the molar ratio of indole to N-benzylidene-4-methylbenzenesulfonamide is 5:1, gives rise to the formation of the 3-indolylmethanamine compound in 80% yield and 83% ee.

Examples 2a-2g show that, unlike the catalysts of the state of the art, the catalysts of the invention allow for the formation of the product in high yield and selectivity, even when a minor excess of indole is used.

### Example 2h: Preparation of (S)-N-((1H-indol-3-yl)(phenyl)methyl)-4-methylbenzenesulfonamide by catalyst recycling

*N*-benzylidene-4-methylbenzenesulfonamide (0.07 mmol), indole (0.1 mmol, 1.4 equivalent) and compound of formula **(IIIh)a** obtained in Example 1 (10 mol%) were placed in a vial and CH₂Cl₂ (0.4 mL, 0.16 M) was added. The reaction mixture was shaken until TLC monitoring showed complete consumption of the starting imine and for the time shown in Table 2. Then, the resin was filtered and the filtrate directly purified by column chromatography on silica gel (from CH₂Cl2 to CH₂Cl₂/EtOAc 96:4).

After each run, the compound **(IIIh)a** was recovered from the filter plate and dried for 2 h under vacuum. Then, the compounds of formula (VIII) and (VII) and CH₂Cl₂ were again added and the same process was repeated successively.

In total, 14 successive runs have been carried out. Table 2 shows the results obtained for each run.

**Table 2**

| Run | Time (h) | yield (%) | ee (%) |
|---|---|---|---|
| 1 | 2.5 | 81 | 93 |
| 2 | 2 | 90 | 90 |
| 3 | 2.5 | 84 | 91 |
| 4 | 2.5 | 84 | 90 |
| 5 | 2.5 | 88 | 88 |
| 6 | 2.5 | 90 | 87 |
| 7 | 9 | 81 | 76 |
| 8^{a} | 3 | 72 | 98 |
| 9 | 2 | 68 | 96 |
| 10 | 1 | 93 | 92 |
| 11 | 2 | 89 | 93 |
| 12 | 2 | 94 | 91 |
| 13 | 2 | 90 | 89 |
| 14 | 2 | 91 | 90 |

| | | | |
|---|---|---|---|
| ^{a} The compound of formula (IIIh) was previously washed with HCl/EtOAc | | | |

Example 2h shows that the catalysts of the invention are highly active and reusable and that high turnover numbers (around 120) can be reached upon catalyst recycling. Example 2h also shows that the catalysts of the invention can be easily reactivated via a washing with an acidic solution.

### Examples 2i-2y: Preparation of various 3-indolylmethanamine compounds

General Procedure A (examples 2i-2u and 2y): The compound of formula (VIII) (0.07 mmol, 1 equivalent), the compound of formula (VII) (amount indicated in Table 3) and the compound of formula **(IIIh)a** obtained in Example 1 (loading indicated in Table 3) were placed in a vial and CH₂Cl₂ (0.4 mL, 0.16 M) was added. The reaction mixture was shaken at room temperature until Thin Layer Chromatography monitoring showed complete consumption of the starting compound of formula (VIII) and for the time indicated in Table 3. Then, the compound **(IIIh)a** was filtered and the filtrate directly purified by column chromatography on silica gel (from CH₂Cl₂ to CH₂Cl₂/EtOAc 96:4), unless otherwise stated.

General Procedure B (examples 2v, 2w and 2x): *N*-benzylidenepyridine-2-sulfonamide (0.04 mmol), compound of formula (VII) (0.05 mmol) and compound of formula **(IIIh)a** obtained in Example 1 (loading indicated in Table 3) were placed in a vial and CH₂Cl₂ (0.5 mL, 0.08 M) was added. The reaction mixture was shaken at room temperature until Thin Layer Chromatography monitoring showed complete consumption of the starting compound of formula (VIII) and for the time indicated in Table 3. Then, the resin was filtered and the filtrate directly purified by column chromatography on silica gel (from CH₂Cl₂ to CH₂Cl₂/MeOH 99:1).

Table 3 shows the obtained results.

| Example | (VII) | (VIII) | Amount of **(IIIh)a** in mol% | t (h) | Amount of (VII) in equiv. | yield (%) | ee (%) |
|---|---|---|---|---|---|---|---|
| 2i | 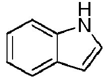 | 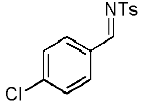 | 10 | 2.5 | 1.4 | 94 | 89 |
| 2j | 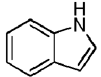 | 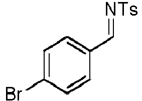 | 10 | 2.5 | 1.4 | 86 | 87 |
| 2k | 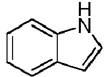 | 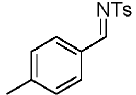 | 10 | 1.5 | 1.4 | 81 | 94 |
| 2l | 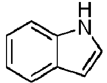 | 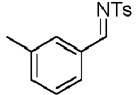 | 10 | 2 | 1.4 | 94 | 91 |
| 2m | 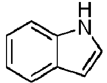 | 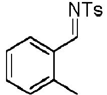 | 10 | 7 | 1.4 | 63 | 85 |
| 2n | 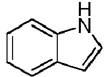 | 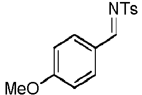 | 10 | 0.8 | 3 | 65 | 98 |
| 2o | 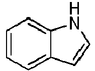 | 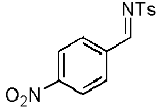 | 10 | 3.5 | 1.4 | 91 | 79 |
| 2p | 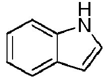 | 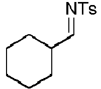 | 10 | 0.5 | 1.4 | 44 | 14 |
| 2q | 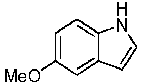 | 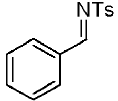 | 10 | 1.5 | 1.4 | 89 | 90 |
| 2r | 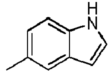 | 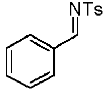 | 10 | 0.75 | 1.4 | 84 | 97 |
| 2s | 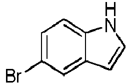 | 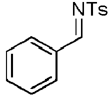 | 10 | 1.5 | 1.4 | 90 | 84 |
| 2t | 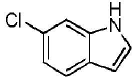 | 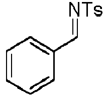 | 10 | 2.5 | 1.4 | 92 | 90 |
| 2u | 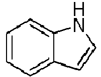 | 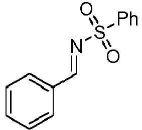 | 10 | 0.75 | 1.4 | 83 | 94 |
| 2v | 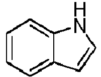 | 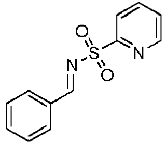 | 8 | 24 | 1.4 | 90 | 90 |
| 2w | 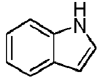 | 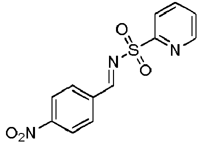 | 8 | 24 | 1.4 | 84 | 77 |
| 2x | 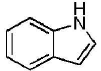 | 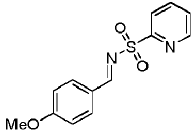 | 8 | 20 | 1.4 | 81 | 83 |
| 2y | 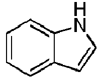 | 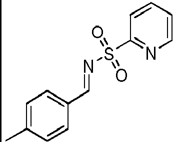 | 8 | 3 | 1.4 | 84 | 84 |

Table 3 shows that the catalysts of the invention are useful in the preparation of a large number of 3-indolylmethanamine compounds and that the presence of additional functional groups does not alter significantly the performance of the catalysts of the invention.

### Example 3: Preparation of 3-indolylmethanamine compounds in continuous flow conditions

### Example 3a: Continuous flow preparation of (S)-N-((1H-indol-3-yl)(p-tolyl)methyl)-4-methylbenzenesulfonamide

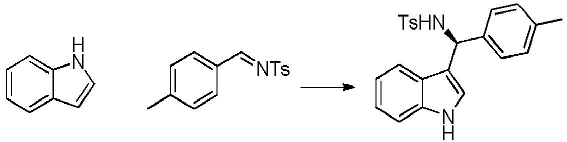

A solution of 4-methyl-*N*-(4-methylbenzylidene)benzenesulfonamide in CH₂Cl₂ (0.32 M) and a solution of indole (0.48 M) in CH₂Cl₂ were pumped through a column loaded with the catalyst **(IIIh)a** prepared by pouring and packing the powdered compound **(IIIh)a** into a ¼ inch Teflon tube of 17 cm length (0.36 g, f = 0.25 mmol·g⁻¹, 0.09 mmol) at a combined flow rate of 0.2 mL·min⁻¹, corresponding to a residence time of 9.3 min. The system was submitted to continuous flow operation for 6 h and the product was collected at the outlet of the reactor. Figure 1 shows the conversion of the compound of formula (VIII) and the enantiomeric excess of the compound of formula (V) as monitored every hour by collecting and analysing samples of the solution exiting of the reactor. Conversion was determined by ¹H NMR spectroscopy and enantiomer excess was determined by chiral HPLC (Chiralcel OD-H column).

Figure 1 shows the evolution over time of the conversion of the compound of formula (VIII) and the enantiomeric excess of the compound of formula (V) produced. As it is shown in Example 3a and Figure 1, the catalysts of the invention allow for the continuous flow production of 3-indolylmethanamine compounds in high yields and selectivity. In this Example, 3.6 g of the 3-indolylmethanamine compound were produced in 6 hours, giving rise to turnover number of 102 and a productivity of 4.3 mmol of the product per hour and gram of catalyst.

### Example 3b: Continuous flow preparation of 3-indolylmethanamines

A solution of a compound of formula (VIII) as shown in Table 4 in CH₂Cl₂ (0.32 M) and a solution of a compound of formula (VII) as shown in Table 4 (0.48 M) in CH₂Cl₂ were pumped through a column loaded with the catalyst **(IIIh)a** prepared as described in Example 3a (0.36 g, f = 0.25 mmol·g⁻¹, 0.09 mmol) at a combined flow rate of 0.2 mL·min⁻¹, corresponding to a residence time of 9.3 min. After one hour, the column loaded with the catalyst **(IIIh)a** was rinsed with CH₂Cl₂ for 30 minutes and solutions of different compounds of formula (VIII) and (VII), as shown in the Table 4 were pumped through the column.

Table 4 shows the yield, enantiomeric excess and productivity, expressed in mmol of the compound of formula (V) per hour and per gram of catalyst of formula (IIIh), obtained with different compounds of formula (VII) and (VIII) following the procedure described above.

**Table 4**

| Compound (VII) | Compound (VIII) | yield (%) | ee(%) | Productivity (mmol.h⁻¹.g⁻¹) |
|---|---|---|---|---|
| 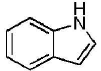 | 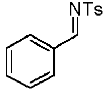 | 87 | 90 | 4.8 |
| 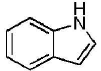 | 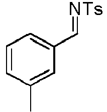 | 94 | 92 | 5 |
| 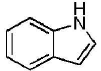 | 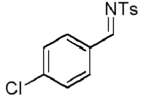 | 86 | 86 | 4.6 |
| 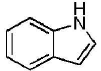 | 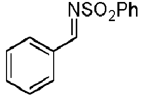 | 92 | 90 | 4.9 |
| 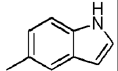 | 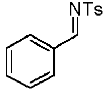 | 80 | 91 | 4.3 |

### REFERENCES CITED IN THE APPLICATION

Kang et al. "Highly Enantioselective Friedel-Crafts Reaction of Indoles with Imines by a Chiral Phosphoric Acid" J. Am. Chem. Soc. 2007, 129, 1484-1485.
Rueping et al. "Synthesis and Application of Polymer-Supported Chiral Bronsted Acid Organocatalysts" Adv. Synth. Catal. 2010, 352, 281-287.
Bleschke et al. "A Chiral Microporous Polymer Network as Asymmetric Heterogeneous Organocatalyst" Adv. Synth. Catal. 2012, 353, 3101-3106.
Wuts et al.,"Greene 's Protective Groups in Organic Chemistry", (Wiley, 4th ed. 2007, p.16-23).

## Claims

1. A chiral compound of formula (I) or a salt thereof 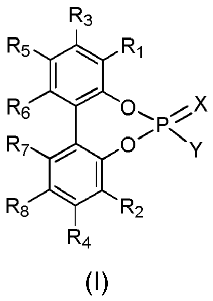 wherein:
X is S or O;
Y is selected from the group consisting of OH and a radical of formula-NHSO₂R₉, wherein R₉ is selected from the group consisting of (C₁-C₆)alkyl, (C₁-C₆)haloalkyl, phenyl and phenyl substituted with one or more groups selected from the group consisting of nitro, halogen and (C₁-C₆)alkyl;
R₁ and R₂ are each independently selected from the group consisting of (C₆-6₂₀ aryl); (C₅-C₂₀)heteroaryl; a radical of formula Si(R₁₀)₃ wherein R₁₀ is selected form the group consisting of phenyl and phenyl substituted with one or more group selected from the group consisting of nitro, halogen, (C₁-C₆)haloalkyl and (C₁-C₆)alkyl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, halogen and a radical of formula (II); and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, halogen and a radical of formula (II);
the radical of formula (II) is 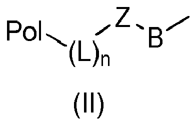 wherein
B and L are each a biradical independently selected from the group consisting of (C₁-C₆)alkylene, phenylene, benzylene, -CH₂-C₆H₄-CH₂- and biphenylene;
n is 0 or 1;
Z is a biradical linked to L and B selected from the group consisting of -O-, -S-, -CONR₁₁-, -SO₂NR₁₁-, -NR₁₁CO-, -NR₁₁SO₂- and -NR₁₁-; wherein R₁₁ is selected from the group consisting of hydrogen, (C₁-C₆)alkyl, phenyl and benzyl;
Pol is a polymeric support;
R₃ and R₄ are each independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen;
each of the pairs R₅ and R₆, and R₇ and R₈, together with the carbon atoms to which they are attached, form a 6-membered carbocyclic ring A and a 6-membered carbocyclic ring A' respectively, said rings being independently saturated, partially unsaturated or aromatic and being optionally substituted with one or more groups selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen,
and the substituent of the position 6 and 6' of the fused ring system formed by the 6-membered ring A or the 6-membered ring A' and the adjacent phenyl ring in the compound of formula (I) is further selected from a radical of formula (II); and
with the proviso that the compound of formula (I) comprises at least one radical of formula (II).

2. The compound according to claim 1, wherein:
R₁ and R₂ are each independently selected from the group consisting of (C₆-C₂₀ aryl); (C₅-C₂₀)heteroaryl; a radical of formula Si(R₁₀)₃ wherein R₁₀ is selected from the group consisting of phenyl and phenyl substituted with one or more group selected from the group consisting of nitro, halogen, (C₁-C₆)haloalkyl and (C₁-C₆)alkyl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, and halogen.

3. The compound according to any of claims 1 or 2, wherein:
R₁ and R₂ are each independently selected from the group consisting of phenyl, naphthyl, anthracenyl, phenantrenyl, biphenylyl, phenylylnaphthalene, furyl, pyridyl, thiophenyl, pyrrolyl, indolyl, benzofuryl, benzothiofuryl, triazolyl, pyrimidyl, quinolyl, isoquinolyl, naphthyridyl and a radical of formula Si(R₁₀)₃ wherein R₁₀ is selected from the group consisting of phenyl and phenyl substituted with (C₁-C₆)alkyl, and wherein the phenyl, naphthyl, anthracenyl, phenantrenyl, biphenylyl, phenylylnaphthalene, furyl, pyridyl, thiophenyl, pyrrolyl, indolyl, indenyl, benzofuryl, benzothiofuryl, triazolyl, quinolyl, isoquinolyl, naphthyridyl and pyrimidyl groups are optionally substituted with one or more groups selected from the group consisting of nitro, (C₁-C₆)alkyl, (C₁-C₆)haloalkyl, phenyl and halogen;
R₃ and R₄ are hydrogen;
X is O; and
Y is -OH or -NHSO₂CF₃.

4. The compound according to any of the claims 1 to 3, wherein the compound of formula (I) is a compound of formula (III) 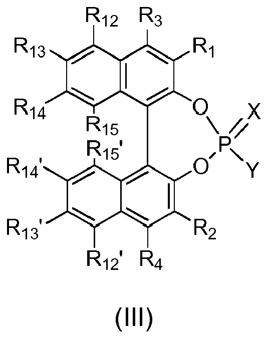 wherein
R₁₂, R₁₄, R₁₅, R₁₂', R₁₄' and R₁₅' are independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen; and
R₁₃ and R₁₃' are independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, halogen and a radical of formula (II) as defined in claim 1; and
with the proviso that one of R₁₃ and R₁₃' is a radical of formula (II).

5. The compound according to claim 4, wherein R₁ and R₂ are the same and R₁₂, R₁₄, R₁₅, R₁₂', R₁₄' and R₁₅' are hydrogen.

6. The compound according to any of the claims 1 to 5, wherein in the radical of formula (II),
L and B are independently selected from the group consisting of methylene, phenylene and benzylene; and
Z is selected from the group consisting of -O- and -SO₂NR₁₁-, wherein R₁₁ is as defined in claim 1.

7. The compound according to any of the claims 4 to 6, wherein the compound of formula (III) is a compound selected from the group consisting of the compounds of formula (IIIa), (IIIb), (IIIc), (IIId), (IIIe), (IIIf), (IIIg), (IIIh) and (IIIi) 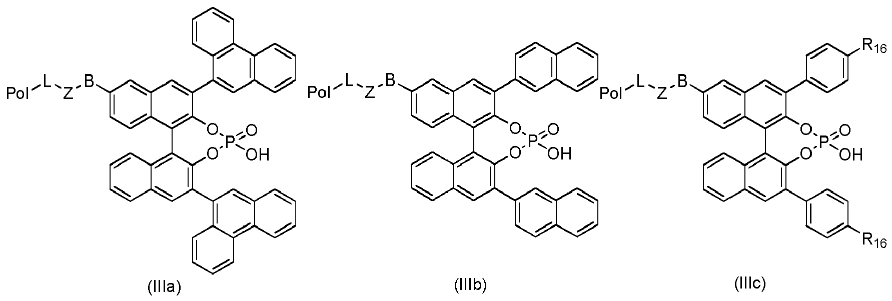 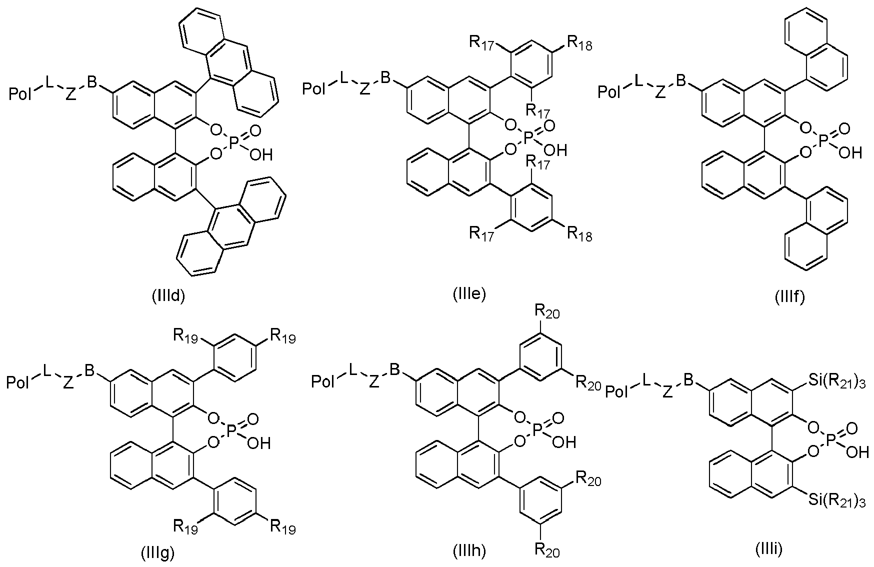 wherein
R₁₆ is a radical selected from the group consisting of hydrogen, nitro, chloro, naphth-2-yl, phenyl, 3,5-(ditrifluoromethyl)phenyl, tolyl, *tert*-butyl, trifluoromethyl and isopropyl;
R₁₇ and R₁₈ are independently selected from the group consisting of hydrogen, methyl, isopropyl, *tert*-butyl, and trifluoromethyl;
R₁₉ and R₂₀ are independently selected from the group consisting of trifluoromethyl and phenyl; and
R₂₁ is selected from the group consisting of phenyl and *tert*-butylphenyl.

8. The compound according to claim 7, wherein
Z is -O-; and
B and L are methylene.

9. The compound according to claim 8, wherein the compound of formula (III) is a compound of formula (IIIh) wherein R₂₀ is trifluoromethyl.

10. A process for the preparation of the compound of formula (I) as defined in any of the claims 1-9, which comprises the steps of:
(i) contacting a polymeric support with a chiral compound of formula (IV) 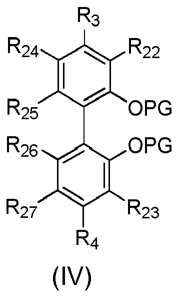
(ii) unprotecting the alcohols on the product obtained in (i);
(iii) treating the product obtained in (ii):
with phosphorus oxychloride when in the compound of formula (I), X is O; or, alternatively,
with thiophosphoryl chloride when, in the compound of formula (I), X is S;
(iv) treating the product obtained in (iii):
with water or alternatively with an acid, when in the compound of formula (I), Y is OH; or, alternatively,
with ammonia, when in the compound of formula (I), Y is a radical of formula-NSO₂R₉; and optionally
(v) when in the compound of formula (I), Y is a radical of formula -NSO₂R₉, then the process further comprises treating the compound obtained in (iv) with a compound of formula R₉SO₂Cl;
wherein:
in the compound of formula (IV):
PG is an alcohol protecting group;
R₂₂ and R₂₃ are independently selected from the group consisting of (C₆-C₂₀ aryl); (C₅-C₂₀)heteroaryl; a radical of formula Si(R₁₀)₃ wherein R₁₀ is selected from the group consisting of phenyl and phenyl substituted with one or more group selected from the group consisting of nitro, halogen, (C₁-C₆)haloalkyl and (C₁-C₆)alkyl; (C₆-C₂₀)aryl wherein one or more of the hydrogen atoms is replaced by one or more group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, halogen and a radical of formula (II'); and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, halogen and a radical of formula (II');
the radical of formula (II') is 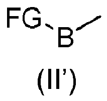
B, R₃ and R₄ are as defined in any of the claims 1 to 9; and wherein
each of the pairs R₂₄ and R₂₅, and R₂₆ and R₂₇, together with the carbon atoms to which they are attached, form a 6-membered carbocyclic ring D and a 6-membered carbocyclic ring D' respectively, said rings being independently saturated, partially unsaturated or aromatic and optionally substituted with one or more groups selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen, and the substituent of the position 6 and 6' of the fused ring system formed by the 6-membered ring D or the 6-membered ring D' and the adjacent phenyl ring in the compound of formula (IV) is further selected from a radical of formula (II'); and with the proviso that the compound of formula (IV) comprises at least one radical of formula (II'); and
wherein:
when in the compound of formula (I), Z is -O-, then the polymeric support comprises at least a terminal halogen group and in the compound of formula (IV), FG is hydroxyl;
or,alternatively,
when in the compound of formula (I), Z is -S-, then the polymeric support comprises at least a terminal halogen group and in the compound of formula (IV), FG is a -SH radical;
or, alternatively,
when in the compound of formula (I), Z is -CONR₁₁-, then the polymeric support comprises at least a terminal group selected from the group consisting of carboxylic acid and carboxylic acid chloride and in the compound of formula (IV), FG is a radical of formula NHR₁₁;
or, alternatively,
when in the compound of formula (I), Z is -NR₁₁CO-, then the polymeric support comprises at least a terminal group of formula NHR₁₁ and in the compound of formula (IV), FG is a group selected from the group consisting of carboxylic acid and carboxylic acid chloride;
or, alternatively,
when in the compound of formula (I), Z is -SO₂NR₁₁-, then the polymeric support comprises at least a terminal sulfonyl chloride group and in the compound of formula (IV), FG is a radical of formula NHR₁₁;
or, alternatively,
when in the compound of formula (I), Z is -NR₁₁SO₂-, then the polymeric support comprises at least a terminal group of formula NHR₁₁ and in the compound of formula (IV), FG is a sulfonyl chloride group;
or, alternatively,
when in the compound of formula (I), Z is -NR₁₁-, then the polymeric support comprises at least a terminal halogen group and in the compound of formula (IV), FG is a radical of formula NHR₁₁; or, alternatively, the polymeric support comprises at least a terminal group of formula NHR₁₁ and in the compound of formula (IV), FG is halogen;
being R₁₁ selected from the group consisting of H, (C₁-C₆)alkyl, phenyl and benzyl.

11. Use of the compound of formula (I) as defined in any of the claims 1 to 9 as a catalyst.

12. A process for the preparation of a 3-indolylmethanamine of formula (XI) 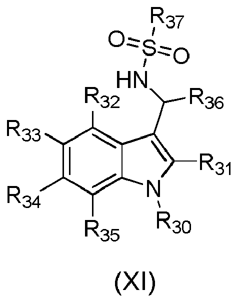 which comprises contacting a compound of formula (IX) with a compound of formula (X) in the presence of a compound of formula (I) as defined in any of the claims 1 to 9 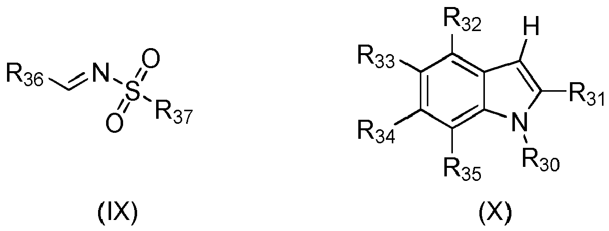 wherein:
R₃₀ is selected from the group consisting of hydrogen, (C₁-C₆)alkyl, (C₁-C₆)haloalkyl, (C₁-C₆)alkylcarbonyl, (C₁-C₆)alkyloxycarbonyl, and benzyl;
R₃₁, R₃₂, R₃₃, R₃₄ and R₃₅ are independently selected from the group consisting of hydrogen, cyclic ring, (C₆-C₂₀)aryl, (C₅-C₂₀)heteroaryl, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, halogen, (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, phenyl, and halogen;
R₃₆ is selected from the group consisting of hydrogen; (C₁-C₆)alkyl; (C₁-C₆)haloalkyl; cyclic ring; (C₆-C₂₀ aryl); (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R₃₇ and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R₃₇ and halogen; and
R₃₇ is selected from the group consisting of (C₁-C₆)alkyl; (C₁-C₆)haloalkyl; cyclic ring; benzyl; (C₆-C₂₀ aryl); (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen;
being cyclic ring a saturated, or partially unsaturated, 3- to 7-membered ring, or 3- to 7- membered ring bridged or fused with one or more 5 to 12 membered ring, saturated, or partially unsaturated, or aromatic, the members of the rings being selected from C, CH, CH₂, O, S, N and NH; being one or more of the hydrogen atoms of the members optionally substituted by a radical selected from the group consisting of (C₁-C₆)alkyl, (C₁-C₆)haloalkyl, halogen, (C₁-C₆)alkoxy, nitro, cyano, (C₁-C₆)alkylcarbonyl, and (C₁-C₆) alkyloxycarbonyl.

13. The process according to claim 12 that is a continuous flow process.

14. The process according to any of the claims 12 or 13, wherein the compound of formula (XI) is a compound of formula (V), the compound of formula (IX) is a compound of formula (VIII), and the compound of formula (X) is a compound of formula (VII), and the process comprises contacting the compound of formula (VII) with the compound of formula (VIII) in the presence of a compound of formula (I); 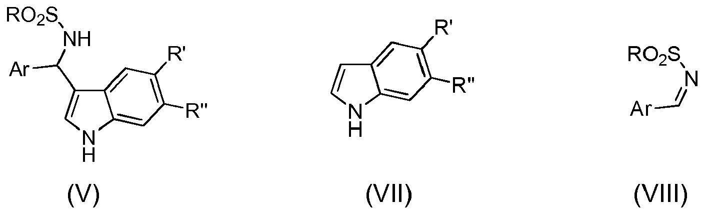 wherein:
Ar is selected from the group consisting of (C₆-C₂₀ aryl); (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R and halogen; and (C₅-C₂₀)heteroaryl wherein one or more of the hydrogen atoms is replaced by a group selected from the group consisting of (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, a radical of formula -CH=NSO₂R and halogen;
R is selected from the group consisting of phenyl, tolyl, fluorenyl, 2-pyridyl, benzyl, and *tert*-butyl; and
R' and R" are independently selected from the group consisting of hydrogen, (C₁-C₆)alkyl, (C₁-C₆)alkyloxy, (C₁-C₆)haloalkyl, nitro, cyano, (C₁-C₆)alkylcarbonyl, (C₁-C₆)alkylcarbonyloxy, (C₁-C₆)alkyloxycarbonyl, and halogen.
15. The process according to any of the claims 12 to 14 wherein the molar ratio of the compound of formula (X) to the compound of formula (IX) is comprised from 1:1 to 3:1 and is carried out at a temperature comprised from 10 °C to 30 °C.
